# EUROPEAN PATENT SPECIFICATION

(11) **EP 1 881 756 B1**
(45) Date of publication and mention of the grant of the patent: **10.08.2016**
(21) Application number: 06734818.5
(22) Date of filing: 10.02.2006
(51) Int. Cl.: A61K 31/132, A61K 31/135, A61K 31/445, A61K 31/55

(54) **TREATING MICROVASCULATURE DISEASES WITH ACETYL CHOLINESTERASE INHIBITORS**
BEHANDLUNG VON KRANKHEITEN DES MIKROVASKULÄREN SYSTEMS MIT ACETYLCHOLINESTERASE-INHIBITOREN
TRAITEMENT DE MALADIES MICROVASCULAIRES AU MOYEN D'INHIBITEURS DE L'ACETYLE CHOLINESTERASE

(30) Priority: 11.02.2005 US 651613 P; 21.03.2005 US 663204 P; 12.04.2005 US 670256 P; 04.05.2005 US 677366 P
(43) Date of publication of application: 30.01.2008
(73) Proprietor: Wills, Stephen, Washington DC 20003 (US)
(72) Inventor: Wills, Stephen, Washington DC 20003 (US)
(74) Representative: Schüssler, Andrea
(86) International application number: PCT/US2006/004857
(87) International publication number: WO 2006/086698

(56) References cited:
- WO-A-2005/102268
- US-A1- 2003 144 255
- PRESS J B ET AL: "RECENT ADVANCES IN OPIOID AND NON-OPIOID ANALGESIA (1992-1993)" CURRENT OPINION IN THERAPEUTIC PATENTS, XX, XX, vol. 4, no. 4, 1 April 1994 (1994-04-01), pages 379-393, XP000892201 ISSN: 0962-2594
- DELAGARZA V: "Pharmacologic Treatment of Alzheimer's Disease: An Update" AMERICAN FAMILY PHYSICIAN, AMERICAN ACADEMY OF FAMILY PHYSICIANS.; US, vol. 68, no. 7, 1 October 2003 (2003-10-01), pages 1365-1372, XP008091098 ISSN: 0002-838x
- TERRY ET AL.: 'The Cholinergic Hypothesis of Age and Alzheimer's Disease Related Cognitive Deficits: Recent Challenges and Their Implications for Novel Drug Development' THE AMERICAN SOCIETY FOR PHARMACOLOGY AND EXPERIMENTAL THERAPEUTICS vol. 306, no. 3, 2003, pages 821 - 827, XP008092584
- DELAGARZA V.: 'Pharmacologic Treatment of Alzheimer's Disease: An Update' AMERICAN FAMILY PHYSICIAN vol. 86, no. 7, 2003, page 2-3 OF 12, XP008091098
- Paul J. Reading ET AL: "Rivastigmine in the treatment of parkinsonian psychosis and cognitive impairment: Preliminary findings from an open trial", Movement Disorders, vol. 16, no. 6, 1 November 2001 (2001-11-01), pages 1171-1174, XP055036031, ISSN: 0885-3185, DOI: 10.1002/mds.1204
- PIRKER W ET AL: 'Cholinesterase-Hemmer in der Therapie der Parkinson-Demenz und der Demenz mit Lewy-Körperchen = Cholinesterase inhibitors in the treatment of Parkinson's disease dementia and dementia with Lewy bodies' JOURNAL FUER NEUROLOGIE, NEUROCHIRURGIE UND PSYCHIATRIE vol. 4, no. 3, 01 January 2003, KRAUSE & PACHERNEGG GMBH, GABLITZ, AT, pages 6 - 10, XP009162146 ISSN: 1608-1587
- D Aarsland ET AL: "Donepezil for cognitive impairment in Parkinson's disease: a randomised controlled study.", J Neurol Neurosurg Psychiatry, vol. 73, no. 3, 1 September 2002 (2002-09-01), page 354, XP055036032,

## Description

### Technical Field of the Invention

The present invention provides a pharmaceutical composition for treating various diseases caused by micro-vasculature circulation problems, including, but not limited to, vascular insufficiency, diabetic neuropathy, inflammatory diseases (e.g., multiple sclerosis, Parkinson's disease), lymphedema, chronic renal insufficiency, and microvascular diseases in visceral organs. Specifically, the inventive pharmaceutical compositionprovides an effective amount of an acetyl cholinesterase inhibitor compound (or combination of compounds) to treat one or a plurality of microvasculature diseases.

### Background of the Invention

Microvascular disease includes a large group of diseases where the pathophysiology is related to the blood flow in the end arterioles-capillaries-venules. This microvascular circulation occurs at the level of the cells and tissues including the vasovasorum of the nerves. To date there has been no effective means of improving blood flow at the microvascular level. In addition to blood flow regulation of the inflammatory cascade from lymphocytes in the past has relied on corticosteroids, methotrexate and more recently TNF (tumor necrosis factor) modulators for control of this process. Leukocyte adhesion that occurs within the microvascular circulation correlates with inflammation and is the clinical entity that correlates with an autoimmune condition. Acetylcholine receptors on lymphocytes provide a means of modulating the inflammatory process by inhibiting the release of cytokines, and pro-inflammatory mediators that produce the cascade of reactions recognized clinically and histologically. The present invention addresses microvascular circulation by improving "run off" and modulates inflammation by influencing the stability of lymphocytes and adhesion of leukocytes to the endothelium within the microvasculature. The impact of treatment with an acetylcholinesterase inhibitor exerts influence upon the parasympathetic nervous system thereby facilitating homestatic functions of the body including blood flow, reduction of pain and inflammation, facilitation of micturition and normal functioning of nerves within the Central Nervous System (CNS) and peripheral nerves. The present invention addresses this need.

Microvasculature diseases include a large group of diseases whose disease etiology originates with problems with white blood cell or leukocyte adhesion in the micro-vasculature. This adhesion of leukocytes causes both vasculature circulation problems, leading to disease symptoms depending on where or which tissue is the site of the reduced blood flow, or an inflammatory cascade of various cytokines and other pro-inflammatory mediators. Various treatments have focused on addressing the downstream effects of microvasculature leukocyte adhesion, such as inhibiting the inflammatory cascade or signaling from such cascade. However, there is a need in the art to address the disease progression through inhibiting an earlier mediator of the disease, before the inflammatory cascade is triggered locally. The present invention addresses this need.

### Diabetes

Diabetes mellitus is implicated in 50-75% of non-traumatic amputations. The etiology of the insult that gives rise to amputation is two fold. The first is the vascular insufficiency on the basis of microvascular disease and the second is neuropathy related to diabetes with the associated loss of sensation. Sensory neuropathy is the primary risk factor for development of diabetic foot ulcers. An estimated 85% of lower extremity amputations occur as a result of sensory neuropathy secondary to diabetes. The presence of peripheral vascular disease is closely linked to diabetes and is associated with compromise of the end arterioles or microvascular circulation. Choline esters have been shown to improve blood flow in skin when administered via ionophoresis. Cholinomimetic agents (acetylcholinesterase inhibitors, choline precursors, and choline esters) have demonstrated the ability to improve circulation in the cortical and subcortical structures of the brain on PET scanning. Acetylcholinesterase inhibitors (e.g., rivastigmine, donepezil) have demonstrated the ability to improve regional blood flow in the brain in both Alzheimer's patients and normal subjects. The use of an acetylcholinesterase inhibitor acts as a cholinomimetic agent by inhibiting the breakdown of acetylcholine thereby improving blood flow through activation of acetylcholine receptors in blood vessels.

Peripheral vascular disease is one of the most common complications of diabetes mellitus. Treatments have included platelet inhibition using aspirin and clopidrogel therapy. Treatment with Pletal® (clostazol) has been fraught with cardiac complications associated with decreased survival and contraindicated in all patients with congestive heart failure of any severity. In severe cases, warfarin is used for anticoagulation to improve perfusion by preventing thrombus formation. Pentoxifylline is used for the purpose of improving the viscosity of blood in patients with chronic peripheral vascular disease. Therapy measures to improve circulation have included exercise to alleviate symptoms of claudication and improve blood flow through collateral circulation. Therapy approaches are limited by compliance and time constraints. To date no effective pharmacological agents have been successful in improving circulation.

A number of surgical solutions have been developed to improve circulation including sympathectomy to remove vascular tone and enhance circulation by decreasing catecholine related vasoconstriction of the end arterioles. The current surgical procedures focus of improving blood flow in large arteries: Aorta, iliac, femoral, tibial and peroneal arteries. The improved blood flow enhances the fluid pressure of the blood impacting the end arterioles. This produces improved circulation but does not address the issue of microcirculation in the skin and distal extremity where gangrene arises from inadequate blood flow to the tissues. This increased pressure leads to vascular and lymphedema with the associated discomfort of chronic swelling in the involved extremity.

One of the first pathological changes in the microvasculature associated with diabetes is a physiological tendency favoring vasoconstriction. This shift toward vasoconstriction diminishes blood flow to the tissue and leads to transudation of fluid into the interstitium producing edema and further exacerbation of tissue ischemia. In addition to vasoconstriction with associated hypertension, pathologic changes occur with the intima of small blood vessels that impair the normal functioning of capillaries or microvascular circulation.

### Acetylcholine

Acetylcholine is the naturally occurring choline ester that is responsible for many neural circuits in human physiology and throughout living systems. This neurotransmitter is perhaps the oldest known neurotransmitter associated with biological systems. The presence of acetylcholine receptors of a non neuronal nature may be responsible for regulation of blood flow within the very small arterioles and ultimately the capillary system which is the critical point for the delivery of oxygen and nutrients to the tissue and cells. The presence of non neuronal acetylcholine receptors within the intima of veins allows for the phenomenon of "run off" to occur which is critical for flow to occur at the microvascular level. Neuronal and non-neuronal receptors for acetylcholine exist within the intima of blood vessels. Non-neuronal acetylcholine receptors have been demonstrated via histochemical analysis of human and mammalian tissue samples. Acetylcholine acts to vasodilate end arterioles decreasing blood pressure and increasing perfusion. In diabetic patients the process of vasoconstriction at the microvascular level introduces tissue ischemia and contributes to the dystrophic changes observed over time, and ultimately the critical ischemia leading to infection and amputation. This tendency for vasoconstriction at the microvascular level is the principle cause of ulceration and tissue necrosis leading to infection from inadequate blood supply. Patients with diabetes develop superficial necrosis in the skin and digits leading to dry gangrene. This dry gangrene frequently progresses to wet gangrene necessitating surgical intervention.

Choline esters have been shown to improve cutaneous blood flow. Methacholine increases cutaneous blood flow and skin temperature in animal models. The mechanism whereby this occurs is thought to be through stimulation of acetylcholine receptor associated with the parasympathetic nervous system. More recent work has demonstrated the presence on non-neuronal acetylcholine receptors within the intima of blood vessels that would contribute to the effects observed in the original studies that were thought to be solely the result of parasympathetic stimulation. Cardiovascular responses include decreased blood pressure through dilitation of small arterioles in addition to lowering of the heart rate. Pulmonary arteriole perfusion has shown improvement with the use of choline esters as well.

Acetylcholinesterase inhibitors are cholinomimetic agents, by virtue of the fact they inhibit the breakdown of acetylcholine through inhibition of cholinesterases and therefore increase acetylcholine activity facilitating cholinergic functions of vasodilitation. Acetylcholinesterases that are associated with the neuromuscular junction are not affected by treatment with acetylcholinesterase inhibitors. Cholinesterases associated with the non-neuronal system of acetylcholine receptors are very sensitive to treatment with acetylcholinesterase inhibitors resulting in sustained acetylcholine related cholinergic physiologic activity.

### Chronic Renal Insufficiency

Chronic renal insufficiency patients often have serum creatinine levels greater than 1.2. Chronic renal insufficiency can have multiple causes including hypertension, diabetes mellitus, medication related (NSAID) and multiple other conditions. Patients with renal insufficiency will manifest progressively increasing values in BUN (blood urea nitrogen) and serum creatinine corresponding to decreasing creatinine clearance. Deterioration in renal function leads to hypertension, congestive heart failure, renal osteodystrophy, acidosis, hyperkalemia and myoneuropathy of renal insufficiency.

Patients with progressive worsening of renal function have diminished cortical blood flow to the kidney and with that loss of blood flow there is a loss of nephrons which constitute the functional units in the kidney responsible for filtration, clearance of nitrogen waste products of metabolism, maintenance of electrolyte balance, regulation of fluid balance and production of erytheopoietin. At creatinine levels of 1.2 or greater these symptoms begin to manifest and require medical management.

### Diabetic Polyneuropathy

Diabetic Polyneuropathy is a common problem facing diabetic patients. It produces sensory and motor complications. The sensory complications include loss of and two-point discrimination loss, vibration and dysesthesias. The motor deficits include atrophy, weakness, tremor and myalgias. The pain component of diabetic polyneuropathy is most troublesome. Ataxia and loss of kinesthetic feedback are common sequelae.

### Lymphedema

Lymphedema is an abnormal accumulation of lymph in the extremities from causes including trauma, burns, surgeries (such as joint replacements), post thrombotic lymphedema, essential lymphedema (Milroy's Disease), and lymphedema from infectious causes resulting in thrombosis of the lymphatic vessels. Current therapeutic approaches include compression garments, sequential pneumatic compression devices; elevation and judicious use of diuretics (furosemide, thiazidediuretic). Long-term sequelae from untreated lymphedema are subject to recurrent attacks of cellulites, with disfigurement and recurrent ulcers leading to surgical excision and skin grafting to maintain the integument.

### Multiple Sclerosis

M.S. is a neurologic condition affecting young adults primarily. It is one of the most common chronic neurologic diseases. The disease is characterized by episodes of focal disorders within the central nervous system. The symptoms remit and recur over a lifetime or present as a progressive demyelinating condition in rare situations. The clinical presentation is dictated by the foci of demyelination with a predilection for certain regions of the central nervous system. Common symptoms include impaired vision, nystagmus, intention tremor, ataxia, bladder dysfunction, limb weakness, emotional liability and paraparesis. At present preventive treatment has included beta interferon for decreasing the likelihood of exacerbations. Treatment of the chronic symptoms has been directed toward specific systems to include Ditropan/Detrol for bladder spasticity, baclofen/dantrolene for spasticity and gabapenin/tricyclic anti-depressants for sensory dysfunction. Steroid treatments are used for acute exacerbations in the form of IV solumedrol pulsed followed by tapering doses of oral preparations.

### Vascular Insufficiency

Vascular insufficiency, particularly in diabetic patients, is related to many factors including: difficulty in controlling infection, large vessel disease necessitating by-pass grafts for revascularization and small arteriolar disease resulting in vascular edema. The overall effect produces an extremity with dystrophic changes, vascular edema and wound healing problems predisposing to gangrene and amputation. Efforts have been directed at prevention of pressure sores and surgical revascularization procedures to improve arterial pressure. There is no current pharmacological therapy or surgical procedure available to improve blood flow in the small arterioles. In the past lumbar sympathectomies have been performed with marginal improvement in clinical outcomes. In theory dilatation of the small arterioles would be possible with the use of parasympathic stimulation via increased concentrations of acetylcholine. Currently medications are available to enhance erectile dysfunction by way of increasing CGMP through phosphodiesterase inhibitors (PDE5). These medications promote vasodilatation in the corpora cavernosa and thereby facilitate penile erection.

The use in peripheral vascular disease would have a similar effect, by promoting dilation of the small arterioles of the extremities, improving vascular flow allowing healing of pressure sores, decreasing vascular edema and improving or eliminating rest pain/claudication symptoms.

### Parkinson's Disease

Parkinson' Disease is condition of the Central Nervous System (CNS) where melanin containing neurons of the brainstem degenerate producing a clinical syndrome clinically recognized as tremors, bradykinesia, and a shuffling gait associated with a stooped posture. The brainstem structures most affected by this disease are the substantia nigra and locus ceruleus. Histochemical studies reflect a decrease amount of dopamine within the caudate nucleus and putamen structures of the basal ganglia. The cause of the disorder is unknown, but there is associated dementia in the later stages of the disease and exacerbation of symptoms with anxiety and tension. The cause of failure of the substantia nigra to occur is speculative with some hereditary predisposition and environmental factors thought responsible. A plausible etiology for degeneration of the substantia nigra relates to compromise of the microvascular circulation within the Central Nervous System supplying these structures.

### Diabetic Polyneuropathy

Treatment of diabetic polyneuropathy is a difficult problem with associated pain, dysfunction, muscle atrophy and motor loss. The most significant problem is one of sensory loss. Pain symptoms are reported as parathesias (abnormal sensations), pain from normal touch (allodynia), and variations of burning, aching or lancinating. The most common symptoms are negative symptoms of sensory loss with inability to feel and perform fine motor manipulation. The problem of inability to judge temperature or feel discomfort in the distal extremities predisposes the patient to injury and ulceration from pressure sores. Sensory neuropathy is the primary risk factor for the development of diabetic foot ulcers that account for the majority of lower extremity amputations. Treatment in the past has included emphasis on meticulous blood glucose control. There is a body literature to support decreased morbidity with improved control of blood glucose. Management of sensory abnormalities has focused on symptomatic treatment with tricyclic antidepressants, gabapentin, and judicious use of narcotics. More recently, serotonin reuptake inhibitors (SSRI's) have been used with marginal success. Their impact on painful diabetic symptoms relates to mood elevation with only theoretical considerations for modulation of endogenous pain pathways.

Treatments have been aimed at palliation of the pain problem with no effective drugs for correcting the atrophy, sensory loss and associated motor dysfunction that can result in foot drop, intrinsic hand wasting, and other syndromes of mononeuropathy multiplex. In addition to hyperglycemia, diabetes has been clearly associated with impairment of the microvascular circulation. Compromise to the microvascular circulation has implications for blood flow to the nerves at the level of the vasovaorum. Interruption of the blood supply to peripheral nerves has a deleterious effect on the function of those nerves and is clearly associated with the pathological process affecting patients with diabetes.

### Diabetes

Diabetes is the leading cause of neuropathy in the Western world. Diabetic neuropathy affects sensory, autonomic and motor nerves of the peripheral nervous system producing dysfunction. Palliative treatment for pain and dysfunction of diabetic polyneuropathy has been the clinical approach for management. Tricyclic antidepressants are thought to influence the perception of pain in the Central Nervous system through preventing re-uptake of serotonin and norepinephrine in the brain. More recently gabapentin, a treatment for partial epilepsy was reported effective in open label series in the treatment of pain syndromes including painful diabetic neuropathy. Gabapentin is structurally related to gamma amino butyric acid (GABA). Gabapentin is not metabolically converted to GABA or an inhibitor of GABA uptake. There several theories for its effect on pain modulation but the actual mechanism is unknown. The mechanism whereby diabetes produces pain is unknown. Serotonin re-uptake inhibitors increase levels of serotonin and norepinephrine influencing endogenous descending pain pathways and thereby inhibiting pain signals and reducing the perception of neuropathic pain. The common feature in tricyclic antidepressants, gabapentin and serotonin re-uptake inhibitors is their modulation of "pain perception". Perception of pain may be related to emotional and psychological conditions that can be improved with the use of medications that enhance mood.

### Acetylcholinesterase Inhibitors

The current treatment armamentarium for Alzheimer's Disease (AD) consists of four cholinesterase inhibitors and an N-methyl-D-aspartate (NMDA) antagonist. The cholinesterase inhibitors that are approved for use in the United States are donepezil (Aricept®), rivastigmine (Exelon®), galantamine (Reminyl®) and tacrine (Cognex®), with the latter being rarely used. Findings of controlled trials demonstrated that the cholinesterase inhibitors preserve levels of acetylcholine in the brain and may provide modest, transient improvement in cognitive and behavioral symptoms (Selkoe and Schenk, 2003). Acetylcholine production declines progressively in AD (Terry and Buccafusco, 2003), eventually reaching the point where cholinesterase inhibition has no benefit (Selkoe and Schenk, 2003). The findings of short-term studies demonstrated that cholinesterase inhibitors are superior to placebo on measures of global and cognitive function in patients with mild-to-moderately severe AD (Rockwood et al., 2001; Rogers et al., 1998; Rösler et al., 1999; Wilcock et al., 2000; Winblad et al., 2001). However, it is not universally agreed that these changes translate into positive outcomes such as maintenance of activities of daily living, reduced caregiver burden and delayed nursing home placement (Clegg et al., 2002; Lanctot et al., 2003).

Acetylcholinesterase inhibitors (such as, donepezil and rivastigmine) have been shown to improve blood flow to CNS structures on PET scanning. Choline esters have demonstrated the ability to improve cutaneous blood flow as well as increase skin temperature when administered via ionophoresis. Acetylcholinesterase inhibitors are cholinomimetic agents inhibiting the break-down of acetylcholine and therefore facilitating cholinergic functions. Treatment with an acetylcholinesterase inhibitor has a general distribution throughout the body as demonstrated in the side effect profile. The net effect of treatment with an acetylcholinesterase inhibitor is to enhance cholinergic functions in the body. The parasympathetic nervous system makes use of cholinergic transmission to facilitate homeostatic function in the body. This results in lowering blood pressure decreasing heart rate and facilitating gastrointestinal transit and many other cholinergic activities related to the parasympathetic nervous system. Neuronal and non-neuronal acetylcholine receptors exist within the intima of blood vessels. This would accounts for the dilitation of blood vessels and lowering of blood pressure associated with administration of acetylcholinisterase inhibitors.

### Summary of the Invention

The present invention provides a pharmaceutical composition for treating diseases associated with micro-vasculature, inflammatory diseases and vascular insufficiency comprising administering an effective amount of an acetylcholinesterase inhibitor. Preferably, the acetylcholinesterase inhibitor is selected from the group consisting of donepezil, galantamine, rivastigmine, combinations thereof, and pharmaceutically acceptable salts thereof. Most preferably, in an adult the daily dosage of donepezil is from about 5 mg to about 10 mg, the daily dosage of galantamine is from about 16 mg to about 32 mg, and the daily dosage of rivastigmine is from about 3 mg to about 9 mg.

The diseases associated with micro-vasculature, autoimmunie/inflammatory diseases, various pain syndromes, urinary retention and vascular insufficiency are selected from the group consisting of chronic renal insufficiency, diabetic polyneuropathy, diabetic peripheral blood flow conditions, diabetic peripheral nervous system conditions, lymphedema, multiple sclerosis, Parkinson's Disease clinical symptoms selected from the group consisting of tremor, hypokinesia and shuffling gait with stooped posture, and combinations of the foregoing disorders.

### Detailed Description of the Invention

The present invention concerns a pharmaceutical composition comprising an acetylcholinesterase inhibitor for use in the treatment by oral administration of chronic renal insufficiency, diabetic polyneuropathy, diabetic vascular insufficiency, lymphedema, multiple sclerosis, and Parkinson's disease clinical symptoms selected from the group consisting of tremor, hypokinesia and shuffling gait with stooped posture.

Preferred embodiments are defined in the dependent claims.

The process of ischemia related to diabetes takes place gradually and relentlessly over time. The incidence of amputation of the contralateral extremity following loss of one limb approaches 50% over the next five years. Patients and physicians are aware of the insidious signs and symptoms that include dystrophic changes in the skin and nails, vascular edema, claudication, and rest pain that occur. Blood flow at the surface level of the skin is most vulnerable. The first appearance of vascular insufficiency is noted in superficial ulceration of the skin. This early sign of ischemia is consistent with microvascular disease and compromise of the very small arterioles and perforators of the skin. Vascular surgical procedures are successful in improving blood flow in the larger arteries. This improved flow increases the fluid pressure that impacts the end arterioles. The improvement in arteriole pressure increases the circulation within the end arterioles and capillaries at the expense of increased edema. The key elements of oxygen and nutrients delivered to the tissue and cells occurs invariably at the microvascular level appears regulated by both neuronal and non-neuronal acetylcholine receptors located in the intima of capillaries and end arterioles. When dilatation of the microvascular circulation is improved delivery of oxygen and nutrients to the cells and tissues occurs with greater facility relieving ischemia. A critical part of the circulation occurs at the microvascular level where exchange of oxygen and nutrients occurs. Simultaneous with delivery of oxygen and nutrients the waste products of metabolism are removed, including carbon dioxide and nitrogenous by products of metabolism. Without being bound by theory or mechanism of action, but the process of vasodilatation of the microvascular circulation appears responsible for the improvement observed in this series of patients. The effect of microvascular circulation occurs in all organ systems and tissues as a result of the end arteriole-capillary relationship that represents the critical point of exchange. The beneficial effect observed in the series of patients discussed herein over a short period of time would likely demonstrate improvement in renal blood flow to the cortex and myocardium as well as organs of digestion where microvascular circulation represents the point in the circulation where exchange of oxygen and nutrients occur. It seems necessary for long term treatment with acetylcholinesterase inhibitors to occur to prevent recurrence of the ischemic problem at the microvascular level.

Patients treated with an acetylcholinesterase inhibitor were noted to have a decrease in vascular edema during treatment. Bernoulli's equation provides an explanation for the physics of fluid mechanics applicable to non-compressible liquids such as blood. The experimental model for this phenomenon can be illustrated in a Venturi tube. This physics model demonstrates the rise in a pressure manometer proximal to a reduction in lumen diameter of a tube relative to the pressure just at or distal to the narrowing of the tube. Applying this model to an end arteriole or capillary this reflects the transudation of fluid as a result of a stenotic blood vessel lumen. When the stenotic restriction is relieved through dilatation of the lumen there is no increased pressure gradient and transudation does not occur. Vasoconstriction of the end arterioles, when relieved by treatment with an acetylcholinesterase inhibitor, produced improved blood and a diminution in the tendency for edema to occur. The phenomenom of edema following revascularization represents a process very similar to the model of the Venturi tube. The same principle might be applied to lymphedema in all patients and in particular following surgical procedures either vascular or orthopedic. The patients faced with the problem of an ischemic limb, when presented with the option of taking an acetylcholinesterase inhibitor for treatment of the underlying problem of compromised microvascular circulation, were skeptical at first and reluctant based on the knowledge that these medications are used for treatment of Alzheimer's Disease. The rationale is difficult for patients to grasp including inherent concerns regarding the mention of Alzheimer's Disease are unsettling. The beneficial effects of treatment are apparent with in days to weeks and any concerns regarding a connection with dementia are quickly assuaged by the dramatic improvement in their vascular insufficiency with alleviation of swelling, pain, resolution of non-healing ulcers with improved motor and sensory function. It is interesting to note that patients have little concern for using a seizure medication for treatment of neuropathic pain.

The potential risks from treatment with an acetylcholinesterase inhibitor are very minimal. The cost saving associated with use of an acetylcholinesterase inhibitor relative to the cost of wound care, and topical agents and nursing care are readily apparent. Patient compliance with treatment was remarkably good and necessary to maintain the integrity of the microvascular circulation. The incidence of adverse side effects was minimal and did not present an obstacle to treatment with an acetylcholinesterase inhibitor.

The problem of microvascular circulation has not been addressed in patients with diabetes and vascular compromise.

In summary, vascular insufficiency from diabetes is associated with compromise of the microvascular circulation. Treatment with an effective dose of an acetylcholinesterase inhibitor raises the relative concentration of acetylcholine promoting vasodilitation of the microcirculation (end arterioles) through stimulation of neuronal and non-neuronal acetylcholine receptors in the intima of the blood vessels. The beneficial effects of promoting vasodilitation and enhanced tissue perfusion correcting the dystrophic changes and alleviating ischemic pain and ulcers is readily apparent in this series of patients. We present a short series of consecutive patents that derived significant improvement in their vascular insufficiency with the use of an acetylcholinesterase inhibitor for treatment of microvascular disease.

### Acetylcholinesterase Inhibitors

Acetylcholinesterase inhibitors increase the amount of neurotransmitter acetylcholine at the nerve terminal by decreasing its breakdown by the enzyme cholinesterase. European Patent 0296560 discloses a number of compounds indicated as acetylcholinesterase inhibitors useful in the treatment of Alzheimer's disease. (1-benzyl-4->(5,6-dimethoxy-1-indanon)-2-yl methylpiperidine, also known as donepezil, E-2020 and ARICEPT) is of particular interest. Suitable doses of the compounds are indicated to be in the range 0.1 to 300 mg, preferably 1 to 100 mg, per adult per day. Examples of active agents for amyloid-related disorders are doxorubicin, galantamine, tacrine (COGNEX), metrifonate, rivastigmine, selegiline, physostigmine, donepezil (ARICEPT), milameline, xanomeline, saeluzole, acetyl-L-carnitine, idebenone, ENA-713, memric, quetiapine, neurestrol and neuromidal. Preferably, the acetylcholinesterase inhibitor or butylcholinesterase inhibitor is selected from donepezil (Aricept), tacrine (Cognex) rivastigmine (Exelon), physostigmine (Synapton), galanthamine (Reminyl), metrifonate (Promem), quilostigmine, tolserine, thiatolserine, cymserine, thiacymserine, neostigmine, eseroline, zifrosilone, mestinon, huperzine A and icopezil or a pharmaceutically acceptable salt of one of the foregoing compounds.

Acetylcholinesterase inhibitors are typically administered as a pharmaceutical composition that comprises (or consists of) the acetylcholinesterase inhibitor that is greater than 95% and preferably greater than 99% pure by weight and one or more excipients, diluents or other inert ingredients commonly found in pharmaceutical compositions. Thus, any acetylcholinesterase inhibitor that are natural products, *i.e.,* produced in nature, are isolated and purified or produced synthetically before being used in the disclosed method.

As used herein, an "effective amount" of a compound of the disclosed invention is a quantity which, when administered to a subject in need of treatment, improves the prognosis of the subject, *e.g.,* delays the onset of and/or reduces the severity of one or more of the subject's symptoms associated with condition being treated. The amount of the acetylcholinesterase inhibitor to be administered to a subject will depend on the particular disease, the mode of administration, the bioavailability of the acetylcholinesterase inhibitor and the characteristics of the subject, such as general health, other diseases, age, sex, genotype, body weight and tolerance to drugs. The skilled artisan will be able to determine appropriate dosages depending on these and other factors. Effective amounts of a pharmaceutically acceptable acetylcholinesterase inhibitor typically ranges between about 0.1 mg/kg body weight per day and about 1000 mg/kg body weight per day, and preferably between 1 mg/kg body weight per day and 100 mg/kg body weight per day.

The route of administration of the acetylcholinesterase inhibitor depends on the condition to be treated. For example, intravenous injection may be preferred for treatment of a systemic disorder such as diabetic polyneuropathy, and oral administration may be preferred to treat a gastrointestinal disorder. The route of administration and the dosage of the acetylcholinesterase inhibitor to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. Thus, depending on the condition, the acetylcholinesterase inhibitor can be administered orally, parenterally, intranasally, vaginally, rectally, lingually, sublingually, bucally, and intrabuccaly to the patient.

Accordingly, acetylcholinesterase inhibitor compositions designed for oral, lingual, sublingual, buccal and intrabuccal administration can be made without undue experimentation by means well known in the art, for example with an inert diluent or with an edible carrier. The compositions may be enclosed in gelatin capsules or compressed into tablets. For the purpose of oral therapeutic administration, the pharmaceutical compositions of the present invention may be incorporated with excipients and used in the form of tablets, troches, capsules, elixirs, suspensions, syrups, wafers, chewing gums and the like.

Tablets, pills, capsules, troches and the like may also contain binders, recipients, disintegrating agent, lubricants, sweetening agents, and flavoring agents. Some examples of binders include microcrystalline cellulose, gum tragacanth or gelatin. Examples of excipients include starch or lactose. Some examples of disintegrating agents include alginic acid, corn starch and the like. Examples of lubricants include magnesium stearate or potassium stearate. An example of a glidant is colloidal silicon dioxide. Some examples of sweetening agents include sucrose, saccharin and the like. Examples of flavoring agents include peppermint, methyl salicylate, orange flavoring and the like. Materials used in preparing these various compositions should be pharmaceutically pure and nontoxic in the amounts used.

Acetylcholinesterase inhibitor compositions of the present invention can easily be administered parenterally such as for example, by intravenous, intramuscular, intrathecal or subcutaneous injection. Parenteral administration can be accomplished by incorporating the cholinergic agonist compositions of the present invention into a solution or suspension. Such solutions or suspensions may also include sterile diluents such as water for injection, saline solution, fixed oils, polyethylene glycols, glycerine, propylene glycol or other synthetic solvents. Parenteral formulations may also include antibacterial agents such as for example, benzyl alcohol or methyl parabens, antioxidants such as for example, ascorbic acid or sodium bisulfite and chelating agents such as EDTA. Buffers such as acetates, citrates or phosphates and agents for the adjustment of tonicity such as sodium chloride or dextrose may also be added. The parenteral preparation can be enclosed in ampules, disposable syringes or multiple dose vials made of glass or plastic.

Rectal administration includes administering the pharmaceutical compositions into the rectum or large intestine. This can be accomplished using suppositories or enemas. Suppository formulations can easily be made by methods known in the art. For example, suppository formulations can be prepared by heating glycerin to about 120 °C., dissolving the acetylcholinesterase inhibitor in the glycerin, mixing the heated glycerin after which purified water may be added, and pouring the hot mixture into a suppository mold.

The present invention includes nasally administering to the subject an effective amount of the acetylcholinesterase inhibitor. As used herein, nasally administering or nasal administration includes administering the acetylcholinesterase inhibitor to the mucous membranes of the nasal passage or nasal cavity of the patient. As used herein, pharmaceutical compositions for nasal administration of a acetylcholinesterase inhibitor include therapeutically effective amounts of the acetylcholinesterase inhibitor prepared by well-known methods to be administered, for example, as a nasal spray, nasal drop, suspension, gel, ointment, cream or powder. Administration of the acetylcholinesterase inhibitor may also take place using a nasal tampon or nasal sponge.

The acetylcholinesterase inhibitor can be administered alone (as a monotherapy) or in combination with one or more other pharmaceutically active agents that are effective against the condition being treated. However, the combination therapy does not include a choline esterase reactivator, as that the term is used in U.S. Pat. No. 5,981,549. For example, they can be administered in combination with an acetylcholine receptor agonist (particularly alpha 7 specific agonists and muscarinic receptor agonists that penetrate the blood brain barrier, see, for example, U.S. Pat. No. 6,610,713 and WO 03/072135 and U.S. Ser. No. 10/729,427, filed Dec. 5, 2003). e.g., anti-microbials, anti-inflammatory agents, analgesics, anti-viral agents, anti-fungals, anti-histamines and the like.

### Microvascular Diseases

### Mrs. R.E.

Mrs. R.E. had undergone right below knee amputation (BKA) secondary to peripheral vascular disease (PVD) and adult onset diabetes mellitus (AODM). Her left foot was cool, mottled with vascular edema. She had an ulcer on the heel posterior medially, approximately 1x 2 cm, non-healing despite treatment with topical antibiotics, colloidal ointment, collagenases and strict pressure relief. The ulcer worsened despite this treatment. No further vascular surgical intervention was possible as reported by the surgeon. Treatment over a prolonged period with coumadin and ASA were unsuccessful. The period of treatment for this condition and vascular impairment was in excess of six (6) months. The patient began a regimen of donepezil 5 mg PO daily. After one month of treatment there was a significant improvement in her temperature and decreased vascular edema. The condition of her heel ulcer was improved from a notable healing edge.

After two months of treatment her vascular edema had trace, the heel ulcer completely resolved. The foot was warm without cyanosis. The nails integrity was clearly improved with near normal appearance. Patient remains on 5 mg donepezil daily without nausea/vomiting or diarrhea symptoms. She became ambulatory with a prosthesis on her amputated limb. This was not possible previously secondary to pain, swelling and vascular insufficiency in her contralateral extremity prior to treatment with an acetylcholinesterase inhibitor for her microvascular disease.

### Mr. D.R.

Mr. D.R. has a history of diabetes mellitus, peripheral vascular disease and left transmetatarsal amputation and right below knee amputation (BKA) converted to right above knee amputation (AKA) with left lower extremity vascular edema and medial malleolar ulcer. There were significant trophic changes consistent with microvascular disease from diabetes mellitus including break down of the surgical incision of the transmetatarsal amputation. Patient was placed on galantamine 4 mg twice daily and titrated upward to 4 mg orally three times. No symptoms of diarrhea or gastroesophageal reflux disease (GERD) were reported. Within two weeks the left lower extremity edema showed significant improvement. The medial malleolar ulcer healed within 4 weeks. Patient had severe phantom pain right AKA site. These symptoms improved dramatically during treatment with galantamine to the point patient did not require narcotics. Additionally patient did not require stool softener or cathartic agents.

### Ms. B.D.

Ms. B.D. had previously undergone right above knee amputation secondary to peripheral vascular disease secondary to diabetes mellitus. Vascular insufficiency in left lower extremity was notable for vascular edema, claudication and trophic changes consistent with peripheral vascular disease (PVD). Her diabetic condition was associated with hypertension. She had been fitted with prosthesis but was limited in functional mobility by pain and swelling in her non amputated lower extremity. Patient was not deemed a candidate for revacularization. She had an ulcer on her heel that limited her ability to walk. Her vascular insufficiency was managed with ASA, 81mg daily. Patient was place on donepezil 5 mg daily after one month of therapy the patient's heel ulcer was resolved. The vascular edema significantly diminished, and patient had no symptoms of claudication. She was pain free. Her ambulatory ability was improved to the point she became a community ambulatory. Previously she had been limited to ambulation only short distances in her home. She experienced no diarrhea or symptoms of gastrointestinal reflux. She remained on 5 mg donepezil daily. Her three month follow-up revealed a warm foot, and near complete resolution of her vascular edema.

### Mr. J.B.

Mr. J.B. underwent left transmetatarsal amputaton with associated left femoral distal by-pass surgery. Despite aspirin therapy, local wound care and antibiotic therapy he succumbed to an left above knee amputation. Concomitant symptoms of vascular insufficiency appeared in his right leg including pain, an ulcer of his right knee and increased vascular edema. The patient carried a diagnosis of adult onset diabetes mellitus and hypertension. He was not considered a prosthetic candidate secondary to his inability to walk with a standard walker. Patient's right foot pain/heal ulcer continued to worsen. He was placed on donepezil 5 mg daily. Within one month his pain complaints were alleviated. The vascular edema in his foot was resolved and the ulcer on his heel markedly improved. After two months of therapy with donepezil 5 mg daily, the patient was ambulatory with walker and the ulcer on his right heel resolved. Patient was considered a candidate for prosthetic fitting and gait training. He was continued on the medical regimen of treatment with an acetylcholinesterase inhibitor for more than one year with no return of vascular insufficiency or complaints of pain from his amputation.

### Ms. W.M.R.

Ms. W.M.R. is an 83-year-old female with history of ESRD (end stage renal disease), insulin dependent diabetes mellitus (IDDM), peripheral vascular disease (PVD), left above knee amputation (AKA) seen in the outpatient setting with rest pain right foot refractory to narcotic pain management. Patient had been excluded as a candidate for revascularization. She had been given trials of ASA (acetylsalicylic acid), tricyclic antidepressants and gabapentin without success. Ms. W.M.R. was place on donepezil 5 mg daily for two weeks. No serious untoward side effects were noted. Her pain was significantly improved, as well as her sleep pattern. The trophic deterioration in her foot improved. The severely atrophic condition of the foot was transformed into viable tissue. She was maintained on this course of treatment for more than six months without any untoward side effects.

### Mr. M.M.

Mr. M. M. is a 64 year old male with history right below knee amputation (BKA), secondary PVD, adult onset diabetes mellitus (AODM), hypertension, with pain and swelling in the non-amputated left lower extremity with symptoms of claudication during gait with the use of a prosthesis in the left leg. His foot was puffy, swollen with vascular edema. Capillary refill was judged as poor. Mr. M.M. was placed on donepezil 5 mg daily for one month with complete resolution of edema in the foot and toes. The temperature of the foot was improved to warm with good capillary refill. His pain was relieved without deleterious side affects such a diarrhea or acid reflux from treatment with an acetylcholinesterase inhibitor.

### Mr. L.W.

Mr. L.W. is a 48-year-old male with history of insulin dependent diabetes mellitus (IDDM), peripheral vascular disease (PVD) and right BKA (below knee amputation). Mr. L.W. had vascular edema in his non-amputated left lower extremity with multiple digital amputations, charcot foot with ulcers on the medial and lateral aspects of the foot and ankle. Mr. L.W. was placed on galantamine 4 mg twice daily and titrated upward to mg 8 mg orally three times daily. The vascular edema in the left leg improved dramatically within one month. The ulcers on the distal third of his left leg resolved over a two-month period. The charcot foot deformity was unchanged. No new ulcer developed during treatment with the acetylcholinesterase inhibitor galantamine. His ambulatory status improved allowing him to walk with single point cane. The treatment time course was 6 weeks in duration. He continued use of galantamine over the next twelve with return of signs or symptoms of vascular insufficiency. There were no untoward side effects from treatment related to pulmonary, gastrointestinal or genitourinary.

### Mr. R.H.

Mr. R.H. is a 58 year old male with a history of diabetes mellitus and a right below knee amputation secondary to peripheral vascular disease. He was compliant with all treatments for dystrophic ulcers on his contralateral extremity from vascular insufficiency. He had multiple ulcers with significant vascular edema on the non-amputated limb despite treatment with topical applications for healing his ulcers and diuretic therapy for his edema. The patient also suffered from chronic renal insufficiency with a creatinine of 1.8. He began a regimen of donepezil 5 mg orally daily. After one month of treatment he had notable healing of his ulcers located on the middle to distal third of his leg with reduction in his swelling. His diuretic therapy was discontinued and the dose of donepezil was increased to 10 mg daily in the absence of any adverse side effects from treatment with an acetylcholinesterase inhibitor. After an additional one month of treatment there was near complete healing of the leg ulcers and complete resolution of his edema. His creatinine normalized to 0.8. He was maintained of this treatment regimen without incident for more than 6 months. Pain issues associated with phantom discomfort in the below knee amputation were abated.

Acetylcholine is the naturally occurring choline ester responsible for mediating vasodilatory effects through the parasympathetic nervous system. The vasodilatory effects mediated through the non-neuronal cholinergic system are likely very significant as a result of the prolonged effect of cholinesterase blockage by acetylcholinesterase inhibitors. By reducing resistance to flow at the end arterioles, edema is eliminated or significantly reduced and perfusion improved.

The improvement in blood flow in the microvascular circulation by acetylcholinesterase inhibitors appears to be dose related. Higher doses have a greater effect by inhibiting the breakdown of acetylcholine, and facilitating dilation of the end arterioles, capillaries and venules or microvascular circulation. Without being bound by theory, theoretical considerations for reduction in vascular edema could be explained on the basis of Bernoulli's equation and its relationship to the Venturi Tube model with the net effect of reducing transudation of fluid into the interstium proximal to the relative constriction of blood vessels further complicating the problem of tissue ischemia. Since parasympathetic nervous innervation via the vagus nerve occurs within the gastrointestinal tract the potential for symptoms of diarrhea and nausea exists. The use of choline esters for vasodilatation is limited by their GI toxicity and degradation that severely blunts any vasodilatory effect in the periphery. The advantage of an acetylcholineserase inhibitor lies in its GI tolerance and provides an effective means of increasing cholinergic transmission with very limited morbidity.

Metoclopramide (Reglan®) is an anticholinergic agent. Gastrointestinal symptoms are ameliorated or eliminated by treatment with metoclopramide when symptoms of diarrhea or nausea limit the use of an acetylcholinesterase inhibitor. Treatment with acetylcholinesterase inhibitor appears likely a long-term therapy in order to maintain vascular integrity within the microvascular circulation. This medical treatment approach does not eliminate other interventions such vascular surgical correction of major arterial blockage or anti-platelet medications but does provide an effective means of addressing microvascular circulation problems in a non-invasive way with very limited morbidity.

Moreover, in addition to the described patient histories described herein, there were an additional seventeen patients presenting with a microvascular disease who were treated with an acetylcholinesterase inhibitor with successful outcomes. There were no unsuccessful outcomes.

### Chronic Renal Insufficiency

To postpone or avoid the sequelae of chronic renal insufficiency, treatment with acetylcholinesterase inhibitors increase the concentration of acetycholine available to promote blood flow to the renal cortex preventing loss of nephrons and allowing existing renal parenchyma to provide the vital function of the kidney. Just as parasympathetic outflow provide homeostatic neural input to the elimination of function of the intestine, the same parasympathetic innervation provides vasodilatation of the small arterioles supplying the cortex of the kidneys allowing for optimal functioning.

### Ms. P.E.

Ms. P.E. was a 63-year-old female with history of hypertension, SVT (supra-ventricular tachycardia) and osteosarcoma of the right foot s/p below knee amputation. Her admission to rehabilitation facility following surgery revealed chronic renal insufficiency with creatinine of 1.7 and BUN (Blood Urea Nitrogen) of 21. She was taking a Beta Blocker for her SVT. Ms. P.E. was given donepezil 5 mg orally for 4 weeks. Follow up creatinine had improved to 1.1. Patient continued the medication for an additional month and creatinine measured at 0.9. She suffered no untoward side effects from treatment.

### Ms. F.C.

Ms. F.C. was a 64-year-old female with history of vascular insufficiency, right below knee amputation, and who noted on routine laboratory screening to have a BUN of 48 and a creatinine of 1.8. She was treated with donepezil 5 mg orally daily for one month. Follow up BUN/creatinine was 37/1.6 respectively. She had no evidence of metabolic acidosis or hyperkalemia. She was treated for an additional 6 weeks with donepezil 5 mg daily. Follow up creatinine was measured 1.2 while he BUN was decreased to 19. Patient suffered no adverse side effects.

### Mr. J.B.

Mr. J.B. was a 78-year-old male with a history of AODM (adult onset diabetes mellitus) hypertension and peripheral vascular disease. During routine testing of serum electrolytes in an outpatient setting his creatinine was noted to 1.4 with BUN (blood urea nitrogen) of 27. Mr. J.B. was not on diuretic therapy. He was given donepezil 5 mg orally daily for one month. His follow up creatinine measured at 0 .9 and BUN was 12. He suffered no untoward side effects from treatment with donepezil.

### Mr. J.A.

Mr. J.A. was a 51-year-old male with history of NIDDM (non insulin dependent diabetes mellitus) hypertension, PVD (peripheral vascular diseased) and CRI (chronic renal insufficiency) status post open left transmetatarsal amputation. On admission his creatinine was measured at 1.6 with a BUN of 11. Mr. J.A. was given donepezil 5 mg orally daily. Less than two weeks following donepezil treatment his creatinine was measured at 1.3. Follow up creatinine measured two weeks later was 1.0. Mr. J.A. was on no diuretic therapy at the time of treatment or prior to treatment.

### Mr. R.M.

R.M. was a 73-year-old male with history of Chronic Renal Insufficiency (CRI) with creatinine of 1.8 and BUN (blood urea nitrogen) of 32 on no diuretic therapy. He was unable to take NSAID for his arthritis secondary to CRI. Mr. R.M. was started on galantamine 4mg orally three times daily. Follow up creatinine was measured at 1.4 with BUN of 21. For convenience of dosing Mr. R.M. was begun on donepezil 5 mg orally daily and galantamine was discontinued. After one month of therapy follow-up creatinine was 1.0 and BUN was 14. Patient was on no diuretic therapy during the course of treatment.

### Mr. R.H.

Mr. R.H. was a 58-year-old male with history of right below knee amputation with dependent edema and chronic renal insufficiency. His BUN measured at 37 and creatinine at 1.4. He was started on donepezil 5 mg orally for vascular insufficiency in the left leg. After 6 weeks of treatment his BUN was measured at 21 and his creatinine was 1.0. He was on no diuretic therapy. He suffered no untoward side effects from treatment.

### Mr. C.D.

Mr. C.D. was an 82-year-old male admitted to a rehabilitation facility following right total knee arthroplasty. On admission his BUN measured 30 and the creatinine measured 1.8. He was on lisinopril for blood pressure control at 5 mg orally daily. Patient was started on donepezil 5 mg orally daily. Over the course of two weeks his BUN improved to 22 and his creatinine decreased to 1.3. He was maintained on lisinopril daily for blood pressure control. He suffered no untoward side effects from treatment. He was discharged on this medication and follow up BUN measured 1 month later was 19 with a creatinine of 1.1.

### Mr. R.R.

Mr. R.R. was 36-year-old male with history of morbid obesity admitted to a rehabilitation hospital following revision surgery for a non-union of his left femur. His admission BUN was measured at 39 creatinine at 2.8. He was treated with low dose lisinopril for his chronic renal insufficiency to enhance cortical blood flood. This patient had no history of hypertension and was on no diuretic therapy. Patient was started on donepezil 5mg orally daily. Over the course of three weeks his BUN was measured at 26 and creatinine at 1.4.

### Mr. F.C.

Mr. F.C. was a 78-year-old male with history of CRI (chronic renal insufficiency). He was seen following THR (total hip replacement) and noted to have a BUN (blood urea nitrogen) of 35 with a creatinine of 1.7. Mr. F.C. was placed on donepezil 5 mg orally daily for one month. Follow up BUN was measured at 24 with creatinine of 1.4. He continued treatment for an additional 6 weeks. After approximately three months of treatment his BUN (blood urea nitrogen) was measured at 20 with a creatinine of 1.1. He suffered no untoward side effects from treatment with donepezil.

### Diabetic Polyneuropathy

The symptoms of motor and sensory loss of diabetic polyneuropathy are ameliorated by acetylcholinesterase inhibitions by increasing concentration of acetylcholine, the principle neurotransmitter of the parasympathetic nervous system. By augmenting the trophic input of nerves of parasympathic nervous system; pain and dyserethesia are diminished and weakness/atrophy of muscles are diminished. The clinical observation in non-Alzheimer's patients is an improvement in motor and sensory function following treatment with effective doses of an acetylcholinesterase inhibitor. Improved blood flow within the microvascular circulation supplying the vasovasorum of the nerves produces the beneficial effect. By restoring blood flow to the vasovasorum of the nerves ischemic pain is relieved and normal function of the nerve is allowed to occur. The process of vasodilatation of the microvascular circulation is mediated through both neuronal and non-neuronal acetylcholine receptors stimulation by acetylcholine. Enhancement in acetylcholine levels is effected through treatment with acetylcholinesterase inhibitors.

### Mr. D.D.

Mr. D.D. was an 80-year-old male with history of left CVA (cerebrovascular accident) right hemi and admitted to hospital with cellulitus right lower extremity. He had no complaints of pain in his legs but described diabetic polyneuropathy symptoms in his hands, which includes burning, tingling and occasional throbbing. Trials of gabapentin and Deseryl® trazadone in therapeutic doses were not helpful. Mr. D.D. was placed on donepezil 5 mg daily. There was notable improvement in hand pain symptoms and improved sleep pattern and decreased vascular edema and redness control in the extremities were improved. This clinical improvement was attributed to therapy with donepezil.

### Mr. V.S.

Mr. V.S. was a 56-year-old male with history of pancreatitis and bilateral lower extremity Diabetic Polyneuropathy with associated pain. Mr. V.S. underwent right THA (total hip arthroplasty) and was admitted to the hospital with bilateral foot pain right greater than left without vascular edema. There were warm feet bilaterally, with pulses intact. Patient had minimal complaints of right hip post op pain. Despite narcotic treatment for his right hip pain patient complained of symptoms of pain in feet, that is, bilateral foot pain was most problematic. Mr. V.S. was placed on galantamine 4 mg PO bid. There was immediate improvement in pain symptoms. He reported sleeping comfortably after treatment with galantamine.

### Ms. P.M.

Ms. P.M. was a 62-year-old female with history of IDDM (insulin dependent diabetes mellitus), and bilateral below knee amputation. Ms. M. complained of bilateral BKA (below knee amputation) pain and bilateral hand pain. She sustained a mild stroke with minimal left side weakness. Her ability to ambulate has been limited by the pain in her hands and bilateral amputation (Trans-tibial). Patient had been given trials of Oxycontin® (long acting oxycodone) 10mg TID with Perocet® (oxycodone/acetaminophen) and Methadone 20 mg daily. Her pain management included doses of Deseryl® (trazadone) and gabapentin for neuropathic pain. Patient had persistent pain despite pain management. The dose of narcotics was limited secondary to cognitive impairment and lethargy.

Ms. P.M. was placed on donepezil 5 mg daily with marked improvement over 4 weeks. She continues PRN (as needed) narcotics but discontinued gabapentin/tricyclic antidepressants feeling that these drugs were of limited benefit. After 4 weeks the dose of donepezil was increased to 10 mg daily. All narcotic pain meds were discontinued. Patient reported significantly improved sleep pattern. Moreover, Ms. M's bilateral BKA and hand pain were sufficiently diminished to allow prosthetic gait secondary to improved diabetic polyneuropathy management.

In addition to the three patient histories provided herein for diabetic polyneuropathy cases, there were twenty-one additional diabetic polyneuropathy patients who were treated with an acetylcholinesterase inhibitor and had successful outcomes. There were no unsuccessful outcomes.

### Lower Extremety Weakness/with Left Foot Drop, AODM (adult onset diabetes mellitus), Diabetic Polyneuropathy

### Ms. T.T.

Ms. T.T was a 54-year-old female with history of hypertension OA (osteoarthritis), asthma CRI (chronic renal insufficiency), status post lumbar laminectomy and fusion. Her past of operative was complicated by left lower extremity weakness and partial foot drop. The physical exam was most consistent with diabetic polyneuropathy, a diagnosis made by a consulting neurologist. Ms. T.T. has a history of asthma. Her mother (deceased) suffered from Alzheimer disease. The left leg felt heavy 'woody', and patient required a rolling walker for ambulation at home and within a community. Ms. T.T. was given a test dose of galantamine 8 mg and observed in the outpatient department for 45 minutes with no ill side effects as related to her pulmonary status. She was placed on 4 mg galantamine QID and titrated to a dose of 8 mg TID with subjective and objective improvement in left leg strength and approximately 80-85% improvement in Left foot drop, avoiding continued use of ankle foot orthosis.

### Ms. M.L.

Ms. M.L. is a 68-year old female with a hasty of IDDM (insulin-dependent diabetes mellitus) and bilateral below knee amputations (BKA's) secondary to peripheral vascular disease (PVD) and gangrene. Ms. M.L. had pain complaints in her hands and BKA bilaterally. She had failed to show significant improvement with gabapentin and tricyclic antidepressants. Ms. M.L. was treated with galantamine 4mg TID initially with notable improvement and placed on a maintenance dose of 8mg BID.

### Mr. L.S.

Mr. L.S. is a 52-year old male with history of IDDM (insulin-dependent diabetes mellitus), non Hodgkins Lymphoma in remission, S/P right THA (total hip arthroplasty). His hospital course, following joint replacement, was characterized by significant pain complaints despite treatment with narcotics. Mr. L.S. was placed on galantamine 4 mg TID and was subsequently increased to 8 mg TID with much improved pain symptoms.

### Ms. P.M.

Ms. P.M. is a 58-year-old female with history of AODM (adult onset diabetes mellitus), right TKA (total knee arthroplasty), hypertension and Diabetic Polyneuropathy with bilateral foot pain, right greater than left. Treatment with gabapentin, narcotics, and tricyclic antidepressants were unsuccessful. She was treated with galantamine 4 mg TID with notable improvement. The dose was increased to 8 mg TID as a maintenance dose with adequate relief for her pain complaints.

### Ms. J.D.

Ms. J.D. is a 53-year-old female with history of chronic back pain and diabetic polyneuropathy superimposed. She has been treated with gabapentin, tricyclic antidepressants and narcotics with only partial relief. Her systems were abated with a combination long acting narcotics and narcotics for break through pain. The patient was started on a regimen of galantamine 4 mg TID with modest improvement. Her dose was increased to 8 mg BID with improved sleep and decreased pain complaints with a dosing regimen of galantamine 8 mg TID. She was able to eliminate long acting narcotics with only occasional use of short acting narcotics.

### Ms. D.J.

Ms. D.J. is a 68-year old female with history of AODM (adult onset diabetes mellitus), hypertension, s/p removal of toxic modular goiter. She had a prolonged hospital course secondary to respiratory problems, all following surgery that required a tracheostomy. During the course of her acute inpatient rehabilitation she complained of bilateral foot pain consistent with diabetic polyneuropathy with associated weakness in the left ankle dorsiflexors. Ms. D.J. was started on rivastigmine 1.5mg orally bid. She reported improvement in her symptoms. The dose was increased to 3mg twice daily with better control. Ms D.J. experienced no gastric or respiratory adverse side effects from treatment with rivastigmine.

### Mr. C.T.

Mr. C.T. is a 64-year-old male with a history of AODM, peripheral vascular disease s/p left above knee amputation with complaints of "phantom pain" and complaints of diabetic polyneuropathy in the intact leg. He complained of insomnia and reported hand tremors. His phantom and diabetic neuropathic pain were uncontrolled with narcotics, tricyclic antidepressants, and gabapentin. Mr. C.T. was started on galantamine 4 mg orally twice daily and titrated upward to four times daily. He reported less diabetic neuropathic pain and significant reduction in "phantom pain" complaints. Mr. C.T. was placed on a maintenance dose of 8 mg orally tid with no reported "phantom pain", diabetic neuropathic plus discomfort and notable improvement in his sleep pattern and resolution of his hand tremor.

### Ms. V.C.

Ms. V.C. is a 50-year-old female with brittle diabetes mellitus status post lumbar laminectomy and fusion. Her post operation course of treatment was complicated by bilateral foot drop (absent ankle dorsiflexor) and bilateral lower extremity weakness. Her condition improved slowly over one year. Imaging studies immediately following her surgery were negative for spinal cord compression. She was eventually able to ambulate in the community with a walker and a single ankle foot orthosis. During the late summer and fall (2004) Ms. V.C. noted increasing weakness in her lower extremities. She has placed on donepezil 5 mg daily. The patient reported increased strength and coordination over the ensuring 4 weeks without adverse side effects, and continues the medication on a daily basis. She was able to discontinue the use of the ankle foot orthosis and became a community ambulatory without assistive devices.

### Mr. W.T.

Mr. W.T. is a 67-year-old male with history of NIDDM (non insulin dependent diabetes mellitus) ESRD (end stage renal disease) s/p renal transplant who underwent orthopedic surgical repair of a right hip fracture. Patient had a pre-existing history of right sided weakness secondary to stroke, but was ambulatory with a cane prior to the hip fracture. Following his fracture surgical fixation he was admitted to a rehabilitation hospital for therapy. Mr. W.T. had difficulty with mobility secondary to increased weakness in bilateral lower extremities following surgery. After one week of therapy with a poor progress to achieve independent ambulation, he was given donepezil 5 mg orally daily. His ambulatory status improved after one week. Donepezil was withdrawn to ascertain if his improved functional mobility was related to donepezil or length of time from his surgery. There was a progressive decline in his ability and strength over the next 7-10 days. Mr. W.T. had no signs or symptoms of early Alzheimer's disease. The donepezil was restarted with improvement in his strength and endurance.

### Ms. G.S.

Ms. G.S. is a 58-year-old female with history of IDDM (insulin dependent diabetes mellitus) with bilateral lower extremity weakness from diabetic polyneuropathy with bilateral foot drop requiring AFO (ankle foot orthosis). She was seen in the outpatient department with complaints of increased weakness and decreased endurance. Ms. G.S. was started on rivastigmine 1.5mg orally twice daily. After one month of treatment she reported no further deterioration in her strength. In the absence of GI side effects the dose was increased to 3.0 mg orally twice daily. Follow-up visit one month later was notable for improved endurance and strength. The dose of rivastigmine was maintained at 3.0 mg twice daily for an additional two months. She reported much improved ability to ambulate with decreased pain. She was able to discontinue the use of the AFO on her right lower extremity.

### Lymphedema

Treatment with an acetylcholinesterase inhibitor increased the neurotransmitter, acetylcholine that is responsible for activation of the parasympathetic nervous system thereby improving arterial perfusion in the small arterioles. The improvement in perfusion within the small arterioles decreases the hydrostatic pressure at the end arterioles and therefore reduces interstitial fluid accumulation that impedes the return of lymph fluid within the lymphatic vessels. The physics model for this phenomenon makes use of Bernouli's equation and its relationship to a Venturi tube. Hydrostatic pressure proximal to a relative stricture creates a pressure gradient that leads to increased transudation that overwhelms the capacity of the lymphatic system to manage. The vasodilatory effects of the parasympathetic nervous system upon small arterioles produces improved perfusion and lessens the accumulation of lymphedema. A common example of lymphedema occurs following joint replacement surgery with swelling that persists for three to six months and sometimes permanently. Lymphedema following joint replacement surgery slows recovery and creates pain while predisposing to deep vein thrombosis and infection.

### Ms. A.S.

Ms. A.S. is an 82-year old female with history of bilateral lower extremity lymphedema refractory to pneumatic compression treatment, compression stockings and diuretic therapy over a five year period. Ms. A.S. was started on donepezil 5 mg orally daily. She suffered symptoms of diarrhea or nausea. Over the course of three months there were notable improvements in her lymphedema such that she was able to ambulate in the community with a single point cane. Prior to treatment with donepezil she required a walker and was home bound.

### Mr. D.T.

Mr. D.T. is a 76-year old male with history of diabetes mellitus, hypertension and right below knee amputation. He suffered from significant lymphedema in his left leg refractory to compression stocking and diuretic therapy. Mr. D.T. was started on donepezil 5 mg orally daily. After one month of therapy there was significant improvement in his lower extremity swelling allowing him to wear shoes and ambulate in the community more easily.

### Ms. L.T.

Ms. L.T. is a 48-year old female with history of back pain secondary to lumbar stenosis and morbid obesity. She suffered from lymphedema refractory to compression stocking, elevation and thiazide diuretics. She was started on galantamine 4mg orally three times daily and quickly titrated upward to 8 mg three times daily. Over the course of two months there was significant improvement in her lower extremity swelling.

### Mr. H.D

Mr. H.D. is a 53-year old male with history of hypertension and osteoarthritis admitted to a rehabilitation facility following right total hip replacement. He experienced significant lymphedema following his surgery refractory to elevation, compression stockings and retrograde massage. Mr. H.D. was started on rivastigmine 1.5 mg orally twice daily. Over the course of 4 days there was notable improvement in the swelling in the absence of compression stockings. He suffered no ill side effects from treatment with rivastigmine.

### Mr. R.C.

Mr. R.C. is a 62-year old female with history of hypertension and osteoarthritis admitted to a rehabilitation hospital following right total knee replacement. Her post operation course was complicated by pain and significant lymphedema limiting her knee flexion and rehabilitation. She suffered from Irritable Bowel Syndrome as noted in her past medical history. She was started on rivastigmine 1.5 mg twice daily. She noted improvement with three days enabling her rehabilitation program to continue more expediency and decreased pain and swelling.

### Mr. T.F.

Mr. T.F. is a 63-year old male with history of left total knee replacement whose post operation course was complicated by chronic swelling with associated pain despite anti-inflammatory drugs, elevation and compression stockings. Doppler study was negative for deep vein thrombosis. Mr. T.F. was started on donepezil 5 mg orally daily. Over the course of one week there was improvement in his lower extremity lymphedema. Following a return one-month later with swelling similar to his immediate post operating condition. He was restarted on donepezil 5mg orally and over 7-10 days his swelling resolved.

### Ms. N.R.

Ms. N.R. is a 72-year old female with asthma, hypertension and osteoarthritis who underwent right total knee replacement. Upon admission to the rehabilitation facility she had complaints of pain and swelling refractory to narcotics, compression stocking and cryotherapy (ice). Patient was started on galantamine 4mg orally three times daily without compromise of her pulmonary condition. The dose was increased to 8 mg orally three times daily with notable improvement in her swelling and similar reduction in her pain. Upon discharge from the hospital the galantamine was discontinued. Three days following discharge patient requested that the galantamine be restarted to control her swelling and pain complaints.

### Mr. Z.T.

Mr. Z.T. is a 70-year old male seen in the outpatient facility one month following right total knee replacement. The patient had persistent swelling in his right leg despite compression stocking and elevation. Doppler study was negative for deep vein thrombosis. Mr. Z.T. was given galantamine 8mg orally twice daily for one month and seen in follow-up. There was marked improvement in his swelling without the use of compression stocking.

A total of 14 additional patients with lymphedema from total joint replacement were treated with an acetylcholinesterase inhibitor with marked improvement in their swelling and improved functioning of the involved extremity.

A total of 6 patients with dependent swelling of the lower extremity refractory to management with compression stocking and diuretics experienced decreased swelling with the use of acetylcholinesterase inhibitor in the absence of diuretics or compression stockings.

The incidence of adverse side effect was notably absent including theoretical worsening of asthma and irritable bowel syndrome. The overall impression was an improvement in the homeostatic functioning of visceral organs.

### Multiple Sclerosis

The present invention provides an acetylcholinesterase inhibitor to increase the relative concentration of acetylcholinesterase within the central nervous system to improve parasympathetic neural transmission and combat the features of M.S. that often present in a craniosacral distribution. Cranial symptoms include mental status changes, emotional lability as well as optic dysfunction and nystgmus. Cranial nerves that control extracular muscles are also affected. Cranial involvement can also manifest as swallowing problems with frank dysphagia. The sacral manifestations of parasympathic dysfunction relate to bowel and bladder control. The symptoms of spastic bladder, urinary retention and constipation are relieved by treatment with an Acetylcholinesterase inhibitor raising the concentrations of acetylcholine to enhance the neural transmission of the parasympathetic nervous system to facilitate normal bowel and bladder function. The craniosacral manifestations of M.S. are reflected in dysfunctional neural transmission associated with the parasympathetic nervous system and can be ameliorated or corrected by treatment with an acetylcholinesterase inhibitor. Improved blood flow within the vasovasorum of the myelin sheath/nerve is also considered useful in amelioration of MS symptoms. Improved blood flow within the microvascular circulation of the vasovasorum of the nerves of the Central Nervous System as well as modulation of lymphocyte activity preventing the inflammatory cascade is considered critical in the mechanism of action.

### Mr. H.N.

Mr. H.N. is a 52-year-old male with history of hypertension and Multiple Sclerosis for many years with recent exacerbation treated with oral steroids. He was non-ambulatory with complaints of right side weakness and pain. His exam was notable for bilateral lower extremity weakness. Mr. H.N. was switched from 10 mg prednisone orally during his steroid taper to donepezil 5mg orally daily. Mr. H.N. reported immediate relief of his pain complaints. The weakness in his legs improved to the point he was able to perform transfers and stand with the aid of parallel bars. He was discharged home at the wheelchair level. Upon return one month later he had persistent weakness in the right hand extensors and was fitted with a volar cock-up splint. In the absence of GI complaints his dose of donepezil was increased to 10 mg orally daily. Follow up exam 6 weeks later was significant for return of anti gravity wrist extension strength. Patient was returned to outpatient therapy for additional gait training owing to improved lower extremity strength and endurance.

### Ms. G.L.

Ms. G.L. is a 55-year-old female with a history of Multiple Sclerosis for the past 20 years. She had a recent exacerbation with bilateral lower extremity weakness, left greater than right. Following pulsed Solumedrol® 1gm daily she was admitted to a rehabilitation hospital for therapy. Her hospital course was notable for increasing left lower extremity weakness, cough with mild dyspnea and spastic bladder. Ms. G.L. was started on galantamine 4mg orally three times daily and quickly titrated upward to 8 mg orally three times daily. She was noted to have increased subjective and objective strength and endurance. Her respiratory and bladder symptoms improved as well.

### Mr. C.A.

Mr. C.A. is a 47-year-old male with a three-year history of Multiple Sclerosis. Diagnosed with lumbar puncture and characteristic findings on head CT scan. He experience bilateral lower extremity weakness and pain with ataxic gait. He was asthmatic by history but not on maintenance drug treatment. He was started on rivastigmine 1.5 mg orally twice daily without exacerbation of asthmatic symptoms, or GI intolerance. The dose was increased to 3mg twice daily with problems. Patient noted improved strength bilateral lower extremity and endurance. He also noted return of erectile function with sexual activity.

### Ms. L.G.

Ms. L.G. is an obese 38-year-old female with history of Multiple Sclerosis for many years. Her most recent exacerbation was treated with IV Solumedrol® over three days with improvement. She was transferred to a rehabilitation facility for therapy with complaints of left side weakness and left esotropia. Ms. L.G. was placed on galantamine 4mg orally three times daily. The dose was well tolerated. Her dose was increased to 8mg three times daily. She had persistent symptoms of left sided weakness. She was switched to donepezil 5 mg daily secondary to GI intolerance from frequent bowel movements. The dosage was increased to donepezil 10mg daily with continued GI tolerance. Her strength gradually improved over two months. Her estropia resolved. The improvement in strength was maintained over 6 month with no further exacerbations.

### Ms. D.A.

Ms. D.A. is a 57-year-old female with history of Multiple Sclerosis treated with Avonex® (interferon beta). CT Scan was consistent with Multiple Sclerosis, as well as clinical documentation of neurological disease. Patient noted unremitting pain in all four extremities in a distal proximal pattern. Pain management has been unsuccessful with combination ant seizure medications, gabapenin, and tricyclic antidepressants in conjunction with the use of narcotics. Ms. D.A. was begun on a regimen of galantamine 4mg orally three times daily and rapidly titrated upward to 8 mg orally three times daily. Her motor symptoms were alleviated, however she still had complaints of hand pain on the finger pads.

### Ms. S.B.

Ms. S.B. is a 32-year-old female with history of Multiple Sclerosis for five years. The presentation of her symptoms includes lower extremity spasticity with bowel and bladder symptoms. Her bowel symptoms are constipation, while her bladder symptoms manifest as a 'spastic bladder' treated with Ditopan® (oxybutinin). She was referred for acupuncture secondary to limited success with Avonex® and Ditropan®. Ms. S.B. was begun on galantamine 4mg orally three times daily. She was seen again in two weeks and reported improvement in her symptoms of bladder spasticity. The dose was titrated upward to 8 mg orally three times daily. Her constipation was relieved as well as her bladder symptoms. Lower extremity spasticity was greatly relieved.

### Ms. J.McF.

Ms. J.McF. is a 73-year-old female with history of Multiple Sclerosis long standing with urinary retention, constipation, and weakness in all four extremities. Her legs were more involved than her arms. She was admitted to a rehabilitation hospital following fall with fracture of her right hip requiring surgical fixation. Upon admission she had an indwelling foley catheter secondary to urinary retention with weakness consistent with her long standing Multiple Sclerosis. Ms. J.McF. was started on galantamine 4 mg orally three times daily. Her foley catheter was removed 48 hours later with post void residuals less that 100 cc's. The dose of galantamine was increased to 8 mg orally three times daily. Over the next two weeks she noted increased strength allowing her to walk. She was unable to ambulate with the same facility prior to admission.

### Mr. K.W.

Mr. K.W. is a 55 y ear old male with history of Multiple Sclerosis with exacerbation resulting bilateral lower extremity weakness left greater than right. Patient had a history of old Polio affecting right leg with a partial foot drop. Mr. K.W. underwent pulsed solumedrol treatment with incomplete recovery. He had persistent weakness in the left lower extremity and bilateral hand tremor. In addition to his motor symptoms he experienced voiding problems associated with urinary urgency. Mr. K.W. was started on galantamine 4mg orally three times daily initially. The dose was increased to 8mg orally three times daily without reported GI or respiratory complications. His bladder symptoms resolved while he gained strength in his left lower extremity and his hand tremor corrected. Mr. K.W. required treatment for 4 weeks. He was then placed on maintenance does of 8 mg orally twice daily with no recurrence of Multiple Sclerosis symptoms over the ensuing three months.

### Polyneuropathy

The present invention further provides a pharmaceutical composition for treating phantom pain following amputation in both upper and lower extremities. "Phantom pain" is a phenomenon described following amputation of either an upper or lower extremity. The sensation is common following amputation and present with varying degrees of discomfort and debility. A patient with phantom pain is typically treated with antidepressants, Neurontin® (gabapeninand) narcotics to a varying degree. The use of acetylcholinesterase inhibitors decreases the need for narcotics and generally eliminates the need for poly pharmacy improving pain, enhancing wound healing and improved sleep pattern in amputee patients. The dose of acetylcholinesterase inhibitor is titrated to GI (gastrointestinal) tolerance with bowel movement consistency and frequency the determining factors. Treatment with acetylcholinesterase inhibitor increases the available acetylcholine which is the neurotransmitter responsible for activation of the parasysmpathic nervous system appear responsible for Noxious symptoms of phantom pain for discomfort. The present invention provides a pharmaceutical composition for treating phantom pain associated with amputation of either upper or lower extremity.

### Mr. C.R.

Mr. C.R. is a 43-year old male with a history of traumatic Left below knee amputation BKA secondary to a motorcycle accident. More than 8 years following his accident the phantom pain in his Left BKA was a persistent problem requiring narcotics on a weekly basis. He had chronic back pain with exacerbation concomitant with his phantom limb pain. Patient was started on galantamine 8 mg BID (twice daily) and subsequently increased to 8 mg TID (thrice daily) with almost complete resolution of his pain.

### Ms. C.L.

Ms. C.L. is 61-year old female with a history of peripheral vascular disease (PVD) secondary to SLE (Lupus). She underwent right below knee amputation (BKA) secondary gangrene. Patient had persistent phantom pain despite treatment with gabapentin and tricyclic antidepressants. The patient had marginal improvement with narcotics but limited their use due to cognitive impairment. Patient was started on donepezil 5 mg daily with dramatic improvement in here phantom discomfort but experienced nausea. She was treated with Reglan® (metoclopramide) and was able to continue treatment with donepezil.

### Mr. D.D.

Mr. D.D. is a 67-year old male with history of vascular disease secondary to tobacco use. He underwent right AKA (above knee amputation. His post operation course was notable for phantom limb discomfort, which responded only to narcotics. However, the narcotics were debilitating, producing lethargy and cognitive impairment. Mr. D.D. was given trials of gabapentin and tricyclic antidepressants without success. He was treated with donepezil 5 mg daily with prompt improvement in his pain control.

### Ms. I.A.

Ms. I.A. is a 54-year old female with history of chronic lower back pain and diabetic in the absence of imaging studies to suggest herniated disc or stenosis. Her pain was refractory to management with narcotics, NSAID's, or tricyclic antidepressants. Patient was placed on galantamine 4 mg orally three times daily with notable improvement in her pain and sleep pattern. Her dose was increased to 8mg orally three times daily with complete resolution in her pain complaints

### Mr. R.H.

Mr. R.H. is a 54-year old male with history of right below knee amputation (BKA) secondary to gangrene from peripheral vascular disease, with similar vascular compromise in his left lower extremity including pre-tibial ulcers. His major complaint is phantom pain in the right BKA site with spasm of the hamstrings refractory to Oxycontin®, tricylic antidepressants and gabapentin. Mr. R.H. was placed on donepezil 5 mg daily for three weeks. His phantom pain diminished to the point he no longer required Oxycotin®. He continued with occasional use of hydrocodone for mechanical pain in the right BKA associated with prosthetic wear.

### Mr. R.T.

Mr. R.T. is a 52-year-old male with history of right above knee amputation AKA secondary to a hypercoaguable state as determined by hematology. His phantom pain complaints have been refractory to management with tricyclic antidepressants, gabapentin, Percocet® and methadone. He has been given a trial of galantamine 8 mg BID over two weeks time. He responded to this treatment by decreasing his use of Oxycodone®/APAP from 5 tablets per day to 2-3 tablets per week.

### Mr. H.F.

Mr. H. F. sustained a left above knee amputation (AKA) in a work related accident. After two months of treatment with Oxycontin® 20 mg TID, and Dildudid® (hydromorphone) 4 mg PO every 4 hours there were no relief, despite concomitant treatment with Trazadone® and gabapentin in therapeutic doses. Patient had no prior history of diabetes or polyneuropathy. Mr. H.F. was treated with galantamine 4 mg BID and titrated to 4 mg four times a day without nausea/vomiting or GI upset. His use of narcotics rapidly discontinued Oxycontin® 10 mg PO BID and significantly reduced the use of Percocet® on a PRN (as needed) basis. Patient continues to report improved sleep and increased energy. Patient had no return of the painful neuropathic pain or phantom discomfort he had previously experienced.

### Mr. M.F.

Mr. M.F. was a young male with traumatic right above knee amputation (AKA) secondary to a pedestrian vs. motor vehicle accident. Initial attempts to control patient pain employed standard treatment with tricyclic antidepressants and gabapentin in therapeutic doses without success, despite liberal use of narcotics including long and short acting. After two months of conventional pain management consisting of tricyclic antidepressants (Trazadone), and narcotics (Oxycontin® 10 mg TID and PRN Perocet®), patient still has significant pain. Patient was placed on galantamine 4 mg QID with improved sleep pattern, complete cessation of long acting narcotics (Oxycontin®) and limited use for PRN medication (Percocet® 2-3/day).

### Mr. T.F.

Mr. T.F. was injured in a work related accident with his left upper extremity severed at the elbow. Patient was referred after six months of pain management that was unsuccessful. Those trials included gabapentin, tricyclic antidepressants and narcotics. Patient was seen initially and placed on standard regimen of Neurontin/Trazadone and PRN narcotics. After 6 weeks, patient reported no improvement in phantom discomfort. Mr. T.F. was on galantamine 4 mg four times daily with dramatic improvement in sleep and phantom pain symptoms. The dose was increased to 8 mg TID without side effects or return of his painful symptoms.

### Mr. T.C

Mr. T.C. is a 78-year old male with history of AODM (adult onset diabetes mellitus), hypertension, peripheral vascular disease status post left above knee amputation secondary to gangrene. Attempts to control his pain with gabapentin, tricyclic antidepressants, and narcotics were unsuccessful. He was placed on donepezil 5 mg daily. Complaints of nausea and vomiting decreased. GI symptoms were reported with treatment with Reglan® (metoclopramide). Donepezil therapy was reinstituted with notable improvement in leg discomfort.

### Ms. S.J.

Ms. S .J. is a 80-year-old female with history of isolated PVD S/P Right below knee amputation (BKA). She has experienced phantom discomfort despite aggressive therapy with gabapentin, tricyclic antidepressants, and narcotics. Her symptom persisted for 3 years following amputation. The patient started treatment with donepezil 5 mg daily. She reported much improved phantom discomfort and improved sleep without nausea and vomiting. She discontinued all other pallitative medications. There were no symptoms of vascular insufficiency in her contralateral extremity over the next 9 months of treatment.

### Vascular Insufficiency

The present invention provides a method for the medical management of peripheral vascular disease. The management of peripheral vascular disease with acetylcholinesterase inhibitor addresses the central dilemma in vascular perfusion of the lower extremities. By increasing the relative concentration of acetylcholine, the parasympthetic nervous system is enhanced, improving blood flow at the tissue level by relaxation of the small end arterioles and improving perfusion therefore decreasing vascular edema, enhancing resolution of trohpic ulcers and alleviating rest pain and claudication complaints. The parasympathetic nervous system controls vascular beds in the small arterioles and by increasing the available acetylcholine, dilatation occurs and blood flow improves. By treatment with acetylcholinesterase inhibitors the end results in improved vascular blood supply, decreased congestion at the small arterioles level and improved blood supply. This is particularly necessary in diabetic patients. The presence of non-neuronal acetylcholine receptors with the intima of blood vessels at the microvascular level represents a second physiologic mechanism whereby dilatation of small blood vessels is facilitated by the use of an acetylcholinesterase inhibitor. This latter mechanism owing to the sensitive nature of the cholinesterases associated with the non neuronal acetylcholine system provides sustained cholinergic activity impacting the microvascular circulation and improving perfusion at the critical end arteriole-capillary-venule level.

### Mrs. R.E.

Mrs. R.E. had undergone right below knee amputation secondary to peripheral vascular disease from AODM (adult onset diabetes mellitus). Her left foot was cool, mottled with vascular edema. She had an ulcer on the heel posterior medially, approximately 1x 2 cm, non-healing despite treatment with topical antibiotics, colloidal ointment, collgeanases and strict pressure relief. The ulcer worsened despite this treatment. No further vascular surgical intervention was possible as reported by the surgeon. Treatment over a prolonged period with coumadin and ASA (acetylsalicylic acid) was unsuccessful. The period of treatment for this condition and vascular impairment was in excess of six (6) months. The patient began a regimen of donepezil 5 mg PO daily. After one month there was a significant improvement in her left foot temperature, and decreased vascular edema. The condition of her heel ulcer was improved from a notable healing edge. After three months of treatment her vascular edema had trace levels and the heel ulcer completely resolved. The foot was warm without cyanosis. The nails demonstrated improved appearance. Patient remains on 5 mg donepezil daily without nausea/vomiting or diarrhea symptoms. She is ambulatory with a prosthesis on her amputated limb. This was not possible previously secondary to pain, swelling and vascular insufficiency.

### Mr. D.R.

Mr. D.R.'s history is diabetes mellitus, peripheral vascular disease, left transmetatarsal amputation and right below knee amputation converted to right above knee amputation with lower extremity vascular edema and medial malleolar ulcer; transmetatarsal amputation ulcer with edema, foot cool. Patient was placed on galantamine 4mg twice daily and titrated upward to 4 mg orally four times daily. No symptoms of diarrhea or gastroesophageal reflux were reported. Within two weeks his left lower extremity edema resolved. The medial malleolar ulcer healed within 4 weeks. Patient had severe phantom pain right above knee amputation site. These symptoms improved dramatically during treatment with galantamine to the point patient did not require narcotics. Additionally patient did not require stool softener or cathartic agents. The patient reported his Bowel function to be normal and much improved as compared to pretreatment condition.

### Ms B.D.

Ms. B.D. had previously undergone right above knee amputation secondary to peripheral vascular disease. Vascular insufficiency in left lower extremity was notable for vascular edema; claudication and trophic changes consistent with peripheral vascular disease. Her diabetic condition was associated with hypertension. She had been fitted with prosthesis but was limited in functional status by pain/swelling in her lower extremity. Patient was not deemed a candidate for revacularization. She had an ulcer on her heel that limited her ability to walk. Her vascular insufficiency was managed with ASA (acetylsalicylic acid), 81 mg daily. Patient was place on donepezil 5 mg daily after 1 month of therapy patient heel ulcer was resolved. The vascular edema significantly diminished, and patient has no symptoms of claudication. She has been pain free. Her ambulatory ability was improved to the point she became a community ambulatory from her prior home ambulatory status. She experienced no diarrhea or ulcer symptoms. She remained on 5 mg donepezil daily. Her three-month follow-up revealed a warm foot, and near complete resolution of her vascular edema.

### Mr. J.B.

Mr. J.B. underwent left TMA (transmetatarsal amputation) with associated left femoral distal by-pass surgery. Despite aspirin therapy, local wound care and antibiotic therapy he succumbed to a leg above knee amputation. Concomitant symptoms of vascular insufficiency appeared in his right leg including pain, an ulcer of his right knee and increased vascular edema. He was not considered a candidate for prosthesis secondary to his inability to walk without prosthesis. Patient's right foot pain/heal ulcer continued to worsen. He was placed on donepezil 5 mg daily. Within one month his pain complaints were alleviated. The vascular edema in his foot was resolved and the ulcer on his heel markedly improved. After two months of therapy with donepezil 5 mg daily, patient was ambulatory with walker, the ulcer on his right heel resolved. Patient was considered a candidate for prosthetic fitting and gait training.

### Ms. W.M.R.

Ms. W.M.R. is an 83-year-old female with history of end stage renal disease on hemodialysis, diabetes mellitus with history of peripheral vascular disease and left above knee amputation seen in the outpatient setting with rest pain right foot refractory to narcotic pain management. Patient has been excluded as a candidate for revascularization. She had been given trial ASA, tricyclic antidepressants without success. Ms. W.M.R was placed on donepezil 5 mg daily for two weeks. No serious untoward side effects were noted. Her pain was significantly improved, as well as her sleep pattern. The trophic deterioration in her foot improved. The near mummification of the foot was transformed into viable tissue. The patient was able to tolerate this treatment for the next 6 months with any adverse side effects related to her diabetic condition, end stage renal disease and the positive changes noted in her right leg and foot persisted without increasing the dose.

### Mr. Mc.M.

Mr. Mc.M. is a 64-year old male with history right below knee amputation secondary peripheral vascular disease, adult onset diabetes mellitus, hypertension, with complaint of pain swelling in left lower extremity and foot and symptoms of claudication with the use of prosthesis for gait in the left leg. His foot was puffy, swollen with vascular edema. Capillary refill was judged as poor. Mr. Mc.M. was placed on donepezil 5 mg daily for one month with complete resolution of edema in the foot and toe. The temperature of the foot was improved to warm. His pain was relieved. No deleterious side effects such a diarrhea or acid reflux were noted. The patient was able to tolerate this medical regimen, and vascular insufficiency noted in the non-amputated limb resolved.

### Mr. L.W.

Mr. L.W. is a 48-year-old male with history of insulin dependent diabetes mellitus, peripheral vascular disease and right below knee amputation. Mr. L.W. had vascular edema left lower extremity with multiple digital amputations, charcot foot with ulcers medial and lateral aspects of lower extremity on the left leg. Mr. L.W. was placed on galantamine 4 mg twice daily and titrated to 4 mg orally four times daily. The vascular edema in the left leg improved dramatically. The ulcers on the distal third of his left leg resolved. The charcot foot deformity was unchanged. No new ischemic ulcers (trophic) developed. His ambulatory status improved allowing gait with single point cane. The treatment time course was 6 weeks in duration.

### Parkinson's Disease

The use of acetylcholinesterase inhibitor enhances microvascular circulation within the CNS. This has been demonstrated on patients with Alzheimer's disease and non Alzheimer's patients on PET scanning. Regional blood flow is increased following administration of donepezil and rivastigmine. I found that treatment of patients with a diagnosis of Parkinson's disease using an acetylcholinesterase inhibitor improved blood flow within the compromised microvascular circulation and restored blood flow to the substantia nigra and locus ceruleus. This increased dopamine levels to the putamen and caudate nucleus. Clinical symptoms of tremors, hypokinesia and shuffling gait with stooped posture were eliminated or abated restoring functional abilities to the patient.

### Ms. R.T.

Ms. R.T. is a 75-year old female with a diagnosis of gait initiation failure (Parkinson's variant) seen in the out patient setting for management of symptoms and therapy recommendations. She complained of "tightness"in her lower extremities and restless leg syndrome. A trial of clonazepam was ineffective in conjunction with physical therapy. The patient had all the symptoms of Parkinson's disease including a resting tremor and hypokinesia. Her left side was more affected by these symptoms but head computerized tomography was negative for cerebrovascular accident. The patient was started on donepezil 5 mg orally daily. After one month of therapy she noted improved function and a decrease in symptoms of restless leg syndrome. She suffered no adverse gastrointestinal symptoms and in fact reported improved bowel function. The dose was increased to 10 mg daily of donepezil with further improvement in her symptoms. She was able to discontinue the use of a walker and was able to ambulate in the community with a single point cane. She developed symptoms of polyuria and frequent bowel movements. These symptoms were treated successfully with the use of Reglan® (metoclopramide) and Detrol® (tolterodine). She continued on this regimen for the next six months without exacerbations or further side effects from treatment with an acetylcholinesterase inhibitor.

### Ms. M.M.

Ms. M.M. is an 80-year old female with a history of Parkinson's disease, hypertension and dementia. Trials of Sinemet® (levadopa/carboxidopa) were poorly tolerated producing negative cognitive changes and nausea. Her dementia was managed with Nemenda® (menantine). Her symptoms of Parkinson's disease and dementia were poorly managed. The patient was admitted to rehabilitation facility for therapy. Upon admission her symptoms were notable for severe hypokinesia, resting tremor and expressionless facial features. The patient was started on donepezil 5 mg orally daily with improvement in her motion disorder and cognition. The dose was titrated upward to 10 mg of donepezil daily with marked improved in her functional mobility and a decrease in tremor and bradykinesia. The major side effect was nocturia that was troublesome to the patient. The dose of donepezil was decreased to 5 mg daily with notable deterioration in her motor function and a return of Parkinsonian symptoms. The patient was started on Detrol LA (tolterodine) 4 mg daily and the dose of donepezil was increased to 10 mg again daily. The patient was able to tolerate this regimen of donepezil 10 mg with Detrol LA 4 mg daily for control of polyuria. She suffered no adverse gastrointestinal symptoms. The patient was discharged home at the ambulatory level using a walker when she had been previously wheelchair bound.

### Mr. A.B.

Mr. A.B. is an 80-year old male with a history of adult onset diabetes mellitus, hypertension, and right below knee amputation. He was diagnosed with Parkinson's disease and transferred to a rehabilitation hospital following right below knee amputation. Upon admission the patient had marked resting tremor in hands and severe bradykinesia. He complained of severe "phantom" pain in his amputated limb and pain in the contralateral extremity consistent with diabetic polyneuropathy. The patient was on hemodialysis secondary to acute on chronic renal failure. Following admission to a rehabilitation facility he was placed on donepezil 5 mg orally daily. The patient noted prompt improvement in his pain related to amputation and diabetic polyneuropathy. Over the next 48 hours there was a notable improvement in his movement disorder with decreased resting tremor and improved mobility. His facial expression became more animated. After one week of treatment with donepezil the dose was increased to 10 mg daily. There was continued improvement in his resting tremor. The bradykinesia was resolved completely. Over the course of 2 weeks the patient began to make urine. His creatinine improved from 5.5 on admission to 4.2 after only two weeks of treatment. The patient complained of abdominal cramps with the increased dose of donepezil. These symptoms were easily managed with the use of metoclopramide 5 mg orally before meals and at bedtime. The patient continued on this medication for the next month with further improvement in his renal function. His creatinine improved to 2.1 and hemodialysis was discontinued. He had no return of Parkinsonian symptoms while on donepezil.

### Mr. M.L.

Mr. M.L. is a 57-year old male with a history of arthritis in his right knee refractory to non-operative management. The patient had a concomitant diagnosis of early onset Parkinson's disease treated with Stalevo® (carbidopa/levadopa/entacapone) 50 mg orally four times daily. The patient underwent total knee arthroplasty on his right knee. Following surgery he was admitted to a rehabilitation facility for comprehensive in patient therapy secondary to his Parkinson's disease and total knee replacement. Upon admission he was noted to have a resting tremor and bradykinesia secondary to Parkinson's disease. There was significant right lower extremity lymphedema secondary to the total knee replacement surgery. The patient's satisfaction with his current Parkinson's therapy was suboptimal. It was agreed to taper the Stalevo® completely in favor of treatment with an acetylcholinesterase inhibitor per discussion with the patient. Mr. M.L. was started on rivastigmine 1.5 mg orally twice daily. Over the next two days he reported improved movement without side effects and less resting hand tremor. The swelling in his leg was improved and the range of motion approaching 90 degrees. After three days the dose of rivastigmine was increased to 3 mg orally twice daily. The patient noted complete resolution of his Parkinson's symptoms. The management with an acetylcholinesterase inhibitor was better tolerated from a gastrointestinal standpoint and the results in terms of rest, tremor, and rigidity were vastly superior to treatment with Stalevo®. The lymphedema in the right leg following total knee replacement was markedly better. The patient was discharged home on 3 mg of rivastigmine twice daily. He suffered no negative side effects for treatment with an acetylcholinesterase inhibitor.

### Diabetic Polyneuropathy

The physiologic shift favoring vasoconstriction that is associated with diabetes is reversed following administration of an acetylcholinesterase inhibitor allowing perfusion of the microvasculature, restoring blood flow to ischemic motor and sensory nerves damaged by the effects of diabetes. The following are a series of consecutive patients with both motor and sensory neuropathy secondary to diabetes mellitus that showed significant improvement with the use of an acetylcholinesterase inhibitor for the treatment of their diabetic polyneuropathy.

### Mr. D.D.

Mr. D.D. is an 80-year old male with history of obesity, insulin dependent diabetes mellitus, Left CVA and right hemiparesis admitted to a rehabilitation facility with right lower extremity cellulitis. He received IV antibiotics for the treatment of the infection. The patient had no complaints of pain in his lower extremities, but complained of burning, tingling and occasionally throbbing pain in his hands bilaterally. There was associated swelling and redness symmetrically in his hands. A trial of gabapentin and trazadone in therapeutic doses was ineffective. Mr. D.D. was given donepezil 5 mg orally at bedtime. There was immediate improvement in his hand pain complaints as well decreased redness and swelling in the hands bilaterally over the next several days. Continued treatment over the next two months occurred without any untoward side effects. There was no return of painful neuropathic symptoms in his hands from diabetic polyneuropathy.

### Mr. V.S.

Mr. V.S. was a 56-year old male with a history of pancreatitis secondary to diabetes mellitus with bilateral lower extremity pain in his feet consistent with diabetic polyneuropathy. The patient was on glargine insulin in the evening and sliding scale coverage for blood sugar control. The patient underwent right total hip replacement and was admitted to a rehabilitation facility for post-operative therapy. His major complaint involved dysesthesias in his feet. The pain in his feet was described as burning with occasional shooting pains. The pain complaints were equally severe on the hip replacement leg as well as the non-operative side. The post operative pain following hip replacement surgery was easily managed with narcotics on a prn schedule. Despite treatment with gabapentin and tricyclic antidepressants he still reported pain symptoms on the plantar aspect of his feet bilaterally. Mr. V.S. was placed on galantamine 4 mg orally three times daily and titrated upward to 8 mg orally three times daily. The patient reported significant improvement in his foot pain and improved sleep pattern. There was a notable reduction in the post-operative lymphedema on the extremity having undergone total hip replacement. There were no associated adverse side effects. He was continued on this drug regimen at the time of discharge and over the next three months without adverse side effects. There was no return of the burning and occasional shooting pain that was associated with his diabetic neuropathy.

### Ms. R.M.

Ms R.M. is a 58-year old female with history of IDDM (insulin dependent diabetes mellitus) and documented diabetic polyneuropathy on electromyographic and nerve conduction velocity studies. Management of her diabetes was accomplished with a split dose of insulin 70/30 twice daily. Her pain symptom worsened and eventually progressed to a state of numbness. Concomitant with the progression of her sensory neuropathy she experienced motor involvement with documented bilateral foot drop. She required a rolling walker and bilateral ankle foot orthoses for ambulation. Ms. R.M. was started on rivastigmine 1.5 mg orally twice daily. After one month she reported improved sensation and return of partial dorsiflexion strength. The dose of rivastigmine was increased to 3.0 mg orally twice daily. F ollow up one month later revealed near complete correction of her foot drop. She was no longer requiring her ankle foot orthoses or rolling walker for ambulation. She suffered no untoward side effects from treatment with an acetylcholinesterase inhibitor. At one point during the course of her treatment she was lost to follow up and allowed her prescription for rivastigmime to lapse. She returned with complaints of weakness and loss of ambulatory ability. She was restarted of rivastigmine and experienced a return of strength and coordination the she had noted with the early drug trial. She has continued the treatment course for more than six months without adverse side effects, or return of dorsiflexion weakness and near normal sensation. There is no evidence of vascular insufficiency or dystrophic changes while she was taking rivastigmine.

### Ms. P.M.

Ms. P.M. is a 62-year old female with a history of IDDM (insulin dependent diabetes mellitus) and bilateral below knee amputations. Her diabetic management included intermediate acting insulin once in the morning. The patient complained of phantom pain in her amputation sites, but had more complaints of burning dysesthesias in her hands bilaterally with redness and swelling consistent with vascular edema. She described the pain as excruciating preventing sleep and interfering with all activities of daily living. Patient had undergone treatment with gabapentin, tricyclic antidepressants and narcotics in therapeutic doses. The rate limiting step for treatment with narcotics were the symptoms of lethargy and cognitive impairment. Ms. P.M. had received doses of methadone as high as 40 mg daily in divided doses with short acting narcotics for breakthrough pain without adequate pain relief. The patient was withdrawn from methadone and placed on donepezil 5 mg orally daily for one month. Her symptoms were improved as compared to treatment with long acting narcotics. In the absence of adverse side effects the dose of donepezil was increased to 10 mg daily. Over the next month there was marked improvement in her pain complaints, both in her hands and amputation sites. She experienced no adverse side effects from treatment with an acetylcholinesterase inhibitor as regards her diabetes, blood pressure or GI tract. She still required occasional short acting narcotics for exacerbation of her pain. The patient tolerated this regimen without any side effects sometimes associated with acetylcholinesterase inhibitors.

### Ms. B.F.

Ms. B.F. is a 48-year old female with insulin dependent diabetes mellitus admitted to an acute care hospital with intractable pain secondary to diabetic polyneuropathy. She had been an insulin dependent diabetic for over five years. She required long and short acting narcotics in conjunction with gabapentin for pain control. She received only minimal amelioration of her pain with this combination of medications. She was subsequently transferred to a rehabilitation hospital to address loss of self care skills and functional mobility secondary to pain complaints and weakness in both upper and lower extremities associated with swelling and redness of the distal portion of both upper and lower extremity. The patient was started on donepezil 5 mg orally daily. She noted prompt improvement in her pain symptoms and decreased swelling in her feet over the next two days. Her doses of long acting narcotics were titrated downward as tolerated. After three days the dose of donepezil was increased to 10 mg daily. All long acting narcotics were discontinued (oxycontin) and she was managed with only hydrocodone on an as needed basis. The swelling in her hands and feet resolved and she was discharge home independent at the ambulatory level with assistive devices. She continued this regimen of donepezil over the next six months without side effect or return of the painful symptoms of diabetic polyneuropathy.

### Ms. J.H.

Ms J.H. is a 61-year old female with a history of IDDM (insulin dependent diabetes mellitus) admitted to a rehabilitation facility following femoral neck fracture treated with open reduction and internal fixation. Patient had suffered from sensory related symptoms of diabetic polyneuropathy with burning on the plantar aspect of her feet. She had no symptoms of claudication by history or vascular insufficiency. During the course of the rehabilitation program her progress became limited by complaints of painful dysesthesias in her feet bilaterally with swelling. Patient was started on donepezil 5 mg orally daily for treatment. She noted prompt improvement in her pain complaints and resolution in pedal edema. The dose was maintained *without* any adverse side effects over the next three months. Her functional status improved as a result of control of pain and swelling in her feet secondary to painful diabetic polyneuropathy.

### Ms. A.R.

Ms A.R. is a 39-year old female with history of insulin dependent diabetes mellitus for ten years. She began to experience weight loss with associated weakness. She became more sedentary and eventually bed bound. During this period she developed a decubitus ulcer on her sacrum and was admitted to an acute care hospital for myocutaneous flap procedure to close the defect. She was receiving glargin insulin and sliding scale coverage for her diabetic management. Following her surgical procedure she was admitted to a rehabilitation post-operative management. During the six-month period of time between the onset of weakness, development of a decubitus ulcer and surgical treatment she had progression of motor loss in both upper and lower extremities consistent with diabetic polyneuropathy documented on electromyographic and nerve conduction studies. Her pain complaints in the lower extremities were severe enough to require narcotics in addition to gabapentin and tricyclic antidepressants. The patient had limited use of the hands secondary to intrinsic hand wasting and marked decrease in her ability to flex the digits of her hands bilaterally. Her lower extremity pain was complicated by foot drop on the left side. She reported severe shooting pain in her feet with atrophy of the musculature. In addition to the motor and sensory neuropathy she had urinary retention having failed several attempts at voiding trials. The patient was started on donepezil 5 mg orally daily for one week. There was immediate reduction in the pain complaints. In the absence of side effects the dose was increased to 10 mg orally daily. Over the next three days there was partial return of ankle dorsiflexion in her left foot. The next voiding trial was successful and patient began to ambulate with an assistive device (rolling walker). She was discharged from the hospital two week off all narcotics. She was pain free and voiding successfully. Her treatment continued over the next 9 months without any adverse side effect. She was able to discontinue the use of the walker within two months. Her dorsiflexion was completely restored in the left foot. The intrinsic wasting in her hand resolved and normal flexion function returned.

Without being bound by theory, the use of an acetylcholinesterase inhibitor, with its ability to improve cholinergic transmission, has the theoretical capability of correcting one of the key elements of diabetic polyneuropathy which is ischemia of the vasovasorum of peripheral nerves both motor and sensory providing a means of addressing the underlying pathology. Every nerve type is vulnerable because all nerves require an adequate vascular supply in order to function properly. There are many manifestations of diabetic neuropathy including diabetic gastroparesis, genitourinary autonomic neuropathy, motor syndromes and sensory dysfunction with pain, and loss of sensation. Sensory motor neuropathy affects large and small afferents nerves. The large afferents control proprioception, cold and vibratory sensation. The small nerves affect pain transmission. The symptoms of sensorimotor progress in a distal to proximal fashion similar to vascular complications associated with diabetes mellitus. There is a well-established correlation between neuronal ischemia and diabetic neuropathy. Microvascular systems are intertwined in the pathophysiology of diabetes and its effect on nerves. This is demonstrated in peripheral vascular disease as well as neuropathy in diabetic patients.

Without being bound by theory, the first pathologic shift in the microvascular system is a tendency favoring vasoconstriction that occurs at the end arterioles of the microvascular circulation leading to diminished blood supply. Ischemia leads to dysfunction manifesting as pain, loss of sensation and vague discomfort or dysesthesias. Acetylcholine receptors have been demonstrated in the intima of blood vessels. There exist both neuronal and non-neuronal acetylcholine receptors. Acetylcholine has been shown to dilate the coronary vessels as well as the pulmonary arterial bed. Acetylcholinesterase inhibitors inhibit the break down of acetylcholine increasing the relative concentration of acetylcholine providing additional neurotransmitter substrate for stimulation of the acetylcholine receptors. By inhibiting the breakdown of acetylcholine, acetylcholinesterase inhibitors act as cholinomimetic agents.

Further, and without being bound by theory, in the microvascular disease progression there is thickening of the basement membrane and endothelial hyperplasia that correlates with the progression of neuronal dysfunction. Cholinesterase activity within the "non-neuronal cholinergic system" occurs at much slower rate than that which exists for the neuronal system therefore allowing for sustained cholinergic activity over a long period with the use of an acetylcholinesterase inhibitor. This sustained cholinergic activity within the non-neuronal system, coupled with effects from the neuronal (parasympathetic) system, may account for improved vasodilitation of the end arterioles and increased blood flow within the microvascular circulation serving peripheral nerves.

Diabetes has been described as a "hypermetobolic" state that promotes elevated intracellular levels of glucose. It is a complex metabolic cascade that leads to the formation of free radicals toxic to the normal function of cells and tissue. Any hypermetabolic state requires additional blood supply to provide the necessary oxygen and nutrients to sustain normal cellular function. The microvascular disease that is the hallmark of diabetes results in blood flow that is inadequate and over time leads to neuronal dysfunction of the motor, sensory and autonomic nerves already compromised by hyperglycemia, free radicals and a stressed metabolic state. A viscous cycle exists between a hypermetabolic state and a compromised microvasular system. Therefore, the use of an acetylcholinesterase inhibitor, by virtue of its cholinomimetic properties, likely promotes vasodilitation in supplying oxygen, and nutrients to compromised nerves.

The cause of diabetic polyneuropathy is not clear. However, internal metabolic pathways are adversely affected by high concentration of intracellular glucose. Rigorous blood sugar control has not been shown to completely prevent or predict the incidence of complication related to diabetes including diabetic polyneuropathy. Glycoxidative and lipoxidative products represent potential deleterious compounds capable of creating pathology leading to polyneuropathy. Protein Kinase C may be a possible source for the aberrant metabolic pathways producing the clinical entity that is recognized by clinicians as diabetic polyneuropathy.

Microvascular disease is associated with the progression of diabetic polyneuropathy. This is demonstrated in histological studies and in animal models indicating that microvascular circulation is compromised early in the disease process and parallels the progression of neuronal dysfunction and the clinical finding observed with diabetic polyneuropathy.

As provided in the present invention, the use of an acetylcholinesterase inhibitor for the treatment of diabetic polyneuropathy has merit based on the cholinomimetic nature of this class of drugs and the clinical cases presented herein, that demonstrate the ability of choline esters to improve blood flow in the skin. Studies have demonstrated improved regional blood flow in the brain with the use of an acetylcholinesterase inhibitor. These finding were observed on PET scanning, but indicate that acetylcholinesterase inhibitors improve blood flow in the brain. The beneficial effects were observed in the thalamus and hippocampus. The systemic effects of acetylcholinesterase inhibitors with the associated lowering of blood pressure and GI motility enhancement indicate that there is general distribution throughout the body and in the periphery.

As provided in the case studies herein, the observation of improved blood flow in the brain appears to occur throughout the body's microvascular circulation. The clinical observations seen in these patients are consistent with improved circulation in the extremities, marked improvement in motor and sensory neuropathy symptoms. The rationale for treatment with an acetylcholinesteraes inhibitor is based on the premise and clinical evidence to impute improved circulation within the microvascular circulation. Actual measurements of microvascular circulation are extremely limited. Improved blood flow at the level of the skin can be inferred by venous plethysmography and transcutaneous oxygen measurements. Improvement in blood flow to motor, sensory, and autonomic nerves can only be appreciated by clinical observation of improved motor function and pain relief. Oral administration of choline ester is poorly tolerated secondary to the direct cholinergic effect on the gastrointestinal system. The major advantage of acetylcholinesterase inhibitors is their tolerance from a gastrointestinal perspective. Techniques for measurement of microvascular to the vasovasorum of the nerve do not exist. Any invasive attempts would serve to disrupt the delicate microvascular system being studied.

The key element of oxygen and nutrients delivered to the tissue and cells occurs at the microvascular level. The control of blood flow at this level appears regulated by both neuronal and non-neuronal acetylcholine receptors and by promoting dilatation of the microvascular circulation improved delivery of oxygen occurs. The critical part of the circulation, exchange of oxygen and nutrients, occurs at the capillary level. Without being bound by theory, the process of vasodilatation at this level appears responsible for the improvement noted in this series of patients. It would appear that improvement in circulation in the vasovasorum of the nerves is not the only area where blood supply at microvascular is affected. It seems very probable that microvascular circulation is improved at the tissue level in all organ systems. This would prove an additional benefit to diabetic patients with renal insufficiency enhancing blood flow to the renal cortex and cardiac patients with cardiovascular autonomic neuropathy improving microvascular blood supply to the myocardium and alleviating ischemia to the heart muscle. Clinical observations of improvement in pain were observed within days, however similar improvement in other organ systems, like renal and cardiac, may require longer duration of treatment to see benefit. It seems that long-term treatment with an acetylcholinesterase inhibitor is necessary to counteract the microvascular complications of diabetes.

Management of diabetic motor dysfunction and painful sensory neuropathy have been difficult in the past with limited goals of palliation. The improvements noted in the case studies provided herein and the theoretical rationale give clinicians an opportunity to improve symptoms and at the same time correct the microvascular problem that contributes to the clinical entity that is recognized as diabetic polyneuropathy and other microvascular diseases. This clinical series offers strong evidence of efficacy based on the ability for acetylcholinesterase inhibitors to act as cholinomimetic agents.

## Claims

1. A pharmaceutical composition comprising an acetylcholinesterase inhibitor for use by oral administration in the treatment of chronic renal insufficiency, diabetic polyneuropathy, diabetic vascular insufficiency, lymphedema, multiple sclerosis, and Parkinson's disease clinical symptoms selected from the group consisting of tremor, hypokinesia and shuffling gait with stooped posture.

2. The pharmaceutical composition for use according to claim 1 wherein the acetylcholinesterase inhibitor is selected from the group consisting of donepezil, galantamine, rivastigmine, combinations thereof, and pharmaceutically acceptable salts thereof.

3. The pharmaceutical composition for use according to claim 1 wherein for an adult the daily dosage of donepezil is from about 5 mg to about 10 mg, the daily dosage of galantamine is from about 16 mg to about 32 mg, and the daily dosage of rivastigmine is from about 3 mg to about 9 mg.

## Patentansprüche

1. Eine pharmazeutische Zusammensetzung umfassend einen Acetylcholinesterase-Inhibitor für die Verwendung, durch orale Verabreichung, bei der Behandlung von chronischer Nierensuffizienz, diabetischer Polyneurophatie, diabetischer vaskulärer Insuffizienz, Lymphödem, Multipler Sklerose und klinischen Symptomen der Parkinson-Krankheit, ausgewählt aus der Gruppe bestehend aus Tremor, Hypokinese und schleppendem Gang mit gebeugter Haltung.

2. Pharmazeutische Zusammensetzung für die Verwendung nach Anspruch 1, wobei der Acetylcholinesterase-Inhibitor aus der Gruppe bestehend aus Donepezil, Galantamin, Rivastigmin, Kombinationen davon und pharmazeutisch verträglichen Salzen davon ausgewählt ist.

3. Pharmazeutische Zusammensetzung für die Verwendung nach Anspruch 1, wobei die tägliche Dosis für einen Erwachsenen von Donepezil von etwa 5 mg bis zu etwa 10 mg reicht, die tägliche Dosis von Galantamin von etwa 16 mg bis etwa 32 mg reicht, und die tägliche Dosis von Rivastigmin von etwa 3 mg bis etwa 9 mg reicht.

## Revendications

1. Composition pharmaceutique comprenant un inhibiteur d'acétylcholinestérase pour utilisation par administration par voie orale dans le traitement d'une insuffisance rénale chronique, d'une polyneuropathie diabétique, d'une insuffisance vasculaire diabétique, d'un lymphoedème, de la sclérose en plaques, et de symptômes cliniques de la maladie de Parkinson choisis dans le groupe constitué par les tremblements, l'hypokinésie et la démarche traînante avec posture voûtée.

2. Composition pharmaceutique pour utilisation selon la revendication 1, dans laquelle l'inhibiteur d'acétylcholinestérase est choisi dans le groupe constitué par le donépézil, la galantamine, la rivastigmine, leurs combinaisons, et leurs sels pharmaceutiquement acceptables.

3. Composition pharmaceutique pour utilisation selon la revendication 1, dans laquelle, pour un adulte, la dose quotidienne de donépézil est d'environ 5 mg à environ 10 mg, la dose quotidienne de galantamine est d'environ 16 mg à environ 32 mg, et la dose quotidienne de rivastigmine est d'environ 3 mg à environ 9 mg.
